# EUROPEAN PATENT SPECIFICATION

(11) **EP 1 123 111 B1**
(45) Date of publication and mention of the grant of the patent: **15.09.2004**
(21) Application number: 99953211.2
(22) Date of filing: 19.10.1999
(51) Int. Cl.: A61K 31/513, A61K 31/706, A61K 31/7068, A61K 31/7125, A61K 48/00, A61P 35/00, C12N 5/10, C12N 15/09

(54) **MODULATION OF DNA METHYLTRANSFERASE EXPRESSION BY COMBINATION THERAPY**
VERÄNDERUNG DER DNS METHYLTRANSFERASE DURCH KOMBINATIONSTHERAPIE
MODULATION DE L'EXPRESSION DE L'ADN METHYLTRANSFERASE PAR THERAPIE COMBINEE

(30) Priority: 19.10.1998 US 104804 P
(43) Date of publication of application: 16.08.2001
(60) Divisional application: 02014370.7
(73) Proprietor: Methylgene, Inc., Saint-Laurent, Quebec H4S 2A1 (CA)
(72) Inventor: BESTERMAN, Jeffrey, M., Montreal, Quebec H9X 3V3 (CA); MACLEOD, Alan, Robert, Westmount, Quebec H3Z 2E8 (CA); SIDERS, William, M., Franklin, Massachusetts 02038 (US)
(74) Representative: Grund, Martin, Dr.
(86) International application number: PCT/US1999/024278
(87) International publication number: WO 2000/023112

(56) References cited:
- WO-A-94/08625
- WO-A-97/32589
- WO-A-97/32604
- JU ET AL: "INCREASED DRUG SENSITIVITY OF TUMOR CELLS BY MANIPULATION OF CELL CYCLE CONTROL GENES" THESIS UNIVERSITY OF SOUTHERN CALIFORNIA,XX,XX, 1 January 1996 (1996-01-01), pages 100-130, XP002081365

## Description

### BACKGROUND OF THE INVENTION

### Field of the Invention

This invention relates to the modulation of gene expression, and to the modulation of mammalian gene expression regulated by methylation.

### Summary of the Related Art

The modulation of expression of genes has long been pursued by researchers. For example, many mammalian diseases are associated with the over- or under-expression of certain genes. By modulating the expression of the gene whose aberrant expression is associated with a disease (or a predisposition to develop such a disease), the disease symptoms may be alleviated.

For example, it would be desirable to modulate the enzyme, DNA Methyltransferase (DNA MeTase), which mediates the methylation of mammalian DNA.

Methylation of mammalian DNA is enzymatically mediated by the covalent modification of the fifth carbon position of the pyrimidine ring of cytosine in CpG dinucleotides. Changes in the pattern of DNA methylation have been correlated with a number of processes in eukaryotes. Holliday (1990) Philos. Trans. R. Soc. Lond. B. Biol. Sci. 326: 329-338 discusses the role of methylation in parental imprinting. Antequera *et al*. (1989) Cell 58: 509-517 discusses the significance of methylation in developmental regulation. Fedoroff *et al*. (1989) Cell 56: 181-191 discloses that methylation is involved in transposition. Hare and Taylor (1985) Proc. Natl. Acad. Sci. USA 82: 7350-7354 discloses that methylation is also implicated in DNA repair. In addition, Gartler and Riggs (1983) Ann. Rev. Genet. 17: 155-190 correlates methylation with X chromosome inactivation, while Bird *et al*. (1986) Nature 321: 209- 213 discloses that methylation also plays a pivotal role in chromatin organization.

While several observations have suggested a role for DNA methylation in cancer pathogenesis, there has been a great deal of disagreement as to the mechanisms involved. Szyf *et al*. (1996) Pharmacol. Ther. 70(1): 1-37 discloses that methylation and demethylation activities are critical components of some tumorigenic growth forms. MacLeod and Szyf (1995) J. Biol. Chem. 270: 8037-8043 discloses that the level of DNA methyltransferase activity may be a nodal control point over oncogenic growth. On the other hand, others have dismissed the theory that DNA Methyltransferase plays a causal role in oncogenesis.

A significant obstacle to the investigation of the role of methylation lies in the deficiencies of available methods to modulate methylation itself. To date, most studies have relied on 5-aza-C and/or 5-aza-dC to inhibit DNA methylation by forming a stable adduct with DNA methyltransferase, thus mimicking the transient covalent intermediate complex believed to be formed during methylation (see, e.g., Wu and Santi (1987) J. Biol. Chem. 262: 4778-4786). This approach, albeit effective in reducing DNA methyltransferase activity and correlating with tumorigenesis inhibition, is therapeutically deficient. Unfortunately, threshold concentrations of 5-azaC or 5-aza-dC empirically necessary to inhibit DNA methyltransferase activity have been found to be toxic to mammals (Juttermann *et al*. (1994) Proc. Natl. Acad. Sci. USA 91: 11797-11801; Laird (1997) Mol. Med. Today 3: 223-229).

More recently, Szyf *et al*. (1995) J. Biol. Chem. 267: 12831-12836, has disclosed a more promising approach to the modulation of DNA methylation using expression of antisense RNA complementary to the DNA methyltransferase mRNA to study the effect of methylation on cancer cells. U.S. Patent No. 5,578,716, discloses the use of antisense oligonucleotides complementary to the DNA methyltransferase gene to inhibit gene expression. These developments have provided powerful new tools for probing the role of methylation in numerous cellular processes. In addition, they have provided promising new approaches for developing therapeutic compounds that can modulate methylation levels.

The effect of antisense inhibition is not immediate, due to the half-life of the DNA methyltransferase enzyme. Thus, although the expression of DNA methyltransferase is modulated, residual enzyme can continue to methylate DNA until such enzyme is degraded. Furthermore, polysome-associated DNA methyltransferase mRNA may also persist for some time, allowing additional translation to produce additional enzyme. In addition, the pharmacodynamic properties of oligonucleotides suggest that lower doses than are currently used could be beneficial (Agrawal *et al*. (1995) Clinical Pharmacokinetics 28: 7-16; Zhang *et al*. (1995) Clinical Pharmacology and Therapeutics 58: 44-53).

Therefore, there remains a need to develop more effective methods for the modulation of the expression of genes, such as DNA methyltransferase, that would reduce the required dosage of antisense oligonucleotides, specific inhibitors of gene products, or other agents currently used while at the same time effectively accomplish the inhibition of gene expression.

### SUMMARY OF THE INVENTION

The present inventors have devised a combination approach by which a gene, such as mammalian DNA methyltransferase (DNA MeTase), is inhibited at the genetic level as well as at the protein level. Surprisingly, this combination approach has been found to reduce the required dosage for efficacy of both antisense oligonucleotides against the gene as well as small molecule inhibitors of the gene product.

The invention also provides improved uses and compositions for the modulation of mammalian DNA MeTase at the genetic level as well as at the protein level and the modulation of mammalian gene expression regulated by methylation. The uses and compositions according to the invention are useful as analytical tools for transgenic studies and as therapeutic tools.

In a first aspect the invention provides a use of an effective synergistic amount of an antisense oligonucleotide which inhibits expression of a gene, and an effective synergistic amount of a protein effector of a product of the gene for the preparation of a pharmaceutical composition for inhibiting the expression of the gene in a cell, wherein the cell is to be contacted with said effective synergistic amounts.

In embodiments of the first aspect of the invention, the cell is to be contacted with an effective synergistic amount of at least one antisense oligonucleotide for an effective period of time. In certain embodiments, the cell is to be contacted with an effective synergistic amount of at least one protein effector for an effective period of time. In certain embodiments, each of the antisense oligonucleotide and the protein effector is admixed with a pharmaceutically acceptable carrier medium prior to contacting the cell. In certain embodiments, the antisense oligonucleotide and the protein effector are mixed prior to contacting the cell.

In certain preferred embodiments of the first aspect of the invention, the cell is to be contacted separately with each of the antisense oligonucleotide and the protein effector. In certain embodiments, the cell is to be contacted with the antisense oligonucleotide prior to being contacted with the protein effector. In certain embodiments, the gene encodes a DNA methyltransferase and the cell to be contacted with the antisense oligonucleotide prior to being contacted with the protein effector is induced to undergo apoptosis or is arrested in the S phase of the cell cycle. In certain embodiments, the cell is to be contacted with the protein effector prior to being contacted with the antisense oligonucleotide. In certain embodiments, the gene encodes a DNA methyltransferase and the cell to be contacted with the protein effector prior to being contacted with the antisense oligonucleotide is arrested in the G, phase of the cell cycle.

In certain preferred embodiments of the first aspect of the invention, the gene encodes a DNA methyltransferase and the cell comprises a gene whose expression has been inactivated by methylation. In certain embodiments, expression of the gene whose expression has been inactivated by methylation is reactivated in the contacted cell. In preferred embodiments, the gene whose expression has been inactivated by methylation is the p16^{ink4} tumor suppressor gene.

In a second aspect, the invention provides a use of an inhibitor for the preparation of a pharmaceutical composition for treating a disease responsive to inhibition of a gene. In the use according to this aspect of the invention a therapeutically effective synergistic amount of an antisense oligonucleotide which inhibits expression of the gene, and a therapeutically effective synergistic amount of a protein effector of a product of the gene is to be administered to a mammal, including a human, which has at least one cell affected by the disease.

In embodiments of the second aspect of the invention, the mammal is to be administered a therapeutically effective synergistic amount of at least one antisense oligonucleotide for a therapeutically effective period of time. In certain embodiments, the mammal is to be ministered a therapeutically effective synergistic amount of at least one protein effector for a therapeutically effective period of time. In certain embodiments, each of the antisense oligonucleotide and the protein effector is admixed with a pharmaceutically acceptable carrier medium prior to administration to the mammal. In certain embodiments, the antisense oligonucleotide and the protein effector are mixed prior to administration to the mammal.

In certain preferred embodiments of the second aspect of the invention, the the antisense oligonucleotide and the protein effector are to be separately administered to the mammal. In certain embodiments, the antisense oligonucleotide is to be administered to the mammal prior to the administration of the protein effector. In certain embodiments, the gene encodes a DNA methyltransferase and the cell affected by the disease in the mammal to which the the antisense oligonucleotide is to be administered prior to the administration of the protein effector is induced to undergo apoptosis or is arrested in the S phase of the cell cycle. In certain embodiments, the protein effector is to be administered to the mammal prior to the administration of the antisense oligonucleotide. In certain embodiments, the gene encodes a DNA methyltransferase and the cell affected by the disease in the mammal to which the protein effector is to be administered prior to the administration of the antisense oligonucleotide is arrested in the G, phase of the cell cycle.

In certain preferred embodiments of the second aspect of the invention, the gene encodes a DNA methyltransferase and the cell affected by the disease comprises a gene whose expression has been inactivated by methylation. In certain embodiments, expression of the gene whose expression has been inactivated by methylation is reactivated in the cell in the mammal to which has been administered a therapeutically effective synergistic amount of an antisense oligonucleotide and a therapeutically effective synergistic amount of a protein effector. In preferred embodiments, the gene whose expression has been inactivated by methylation is the p16^{ink4} tumor suppressor gene.

In a third aspect, the invention provides a use of an inhibitor for the preparation of a pharmaceutical composition for inhibiting tumor growth in a mammal. In the use according to this aspect of the invention a therapeutically effective synergistic amount of an antisense oligonucleotide which inhibits expression of a gene involved in tumorigenesis, and a therapeutically effective synergistic amount of a protein effector of a product of the gene is to be administered to a mammal, including a human, which has at least one neoplastic cell present in its body.

In embodiments of the third aspect of the invention, the mammal is to be administered a therapeutically effective synergistic amount of more than one antisense oligonucleotide for a therapeutically effective period of time. In certain embodiments, the mammal is to be administered a therapeutically effective synergistic amount of at least one protein effector for a therapeutically effective period of time. In certain embodiments, each of the antisense oligonucleotide and the protein effector is admixed with a pharmaceutically acceptable carrier medium prior to administration to the mammal. In certain embodiments, the antisense oligonucleotide and the protein effector are mixed prior to administration to the mammal.

In certain preferred embodiments of the third aspect of the invention, the the antisense oligonucleotide and the protein effector are to be separately administered to the mammal. In certain embodiments, the antisense oligonucleotide is to be administered to the mammal prior to the administration of the protein effector. In certain embodiments, the gene encodes a DNA methyltransferase and the neoplastic cell in the mammal to which the the antisense oligonucleotide is to be administered prior to the administration of the protein effector is induced to undergo apoptosis or is arrested in the S phase of the cell cycle. In certain embodiments, the protein effector is to be administered to the mammal prior to the administration of the antisense oligonucleotide. In certain embodiments, the gene encodes a DNA methyltransferase and the neoplastic cell in the mammal to which the protein effector is to be administered prior to the administration of the antisense oligonucleotide is arrested in the G, phase of the cell cycle.

In certain preferred embodiments of the third aspect of the invention, the gene encodes a DNA methyltransferase and the neoplastic cell comprises a gene whose expression has been inactivated by methylation. In certain embodiments, expression of the gene whose expression has been inactivated by methylation is reactivated in the neoplastic cell in the mammal to which has been administered a therapeutically effective synergistic amount of an antisense oligonucleotide and a therapeutically effective synergistic amount of a protein effector. In preferred embodiments, the gene whose expression has been inactivated by methylation is the p16^{ink4} tumor suppressor gene.

In certain embodiments of the first three aspects of the invention, the gene encodes a DNA methyltransferase. In certain embodiments, the protein effector is selected from the group consisting of 5-aza-cytidine, 5-aza-2'-deoxycytidine, 5-fluoro-2'-deoxycytidine and 5,6-dihydro-5-azacytidine.

In various embodiments of the first three aspects of the invention, the antisense oligonucleotide is in operable association with a protein effector. In certain embodiments, the antisense oligonucleotide has at least one internucleotide linkage selected from the group consisting of phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleotide linkages. In certain embodiments, the antisense oligonucleotide is a chimeric oligonucleotide comprising a phosphorothioate, phosphodiester or phosphorodithioate region and an alkylphosphonate or alkylphosphonothioate region. In certain embodiments, the antisense oligonucleotide comprises a ribonucleotide or 2'-O-substituted ribonucleotide region and a deoxyribonucleotide region.

In a fourth aspect, the invention provides an inhibitor of a gene comprising an antisense oligonucleotide which inhibits expression of the gene in operable association with a protein effector of a product of the gene. In certain embodiments of this aspect of the invention, the antisense oligonucleotide is in operable association with two or more protein effectors.

In certain embodiments of the fourth aspect of the invention, the gene encodes a DNA methyltransferase. In certain embodiments, the protein effector is selected from the group consisting of 5-aza-cytidine, 5-aza-2'-deoxycytidine, 5-fluoro-2'-deoxycytidine and 5,6-dihydro-5-azacytidine.

In certain embodiments of the fourth aspect of the invention, the antisense oligonucleotide has at least one internucleotide linkage selected from the group consisting of phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleotide linkages. In certain embodiments, the antisense oligonucleotide is a chimeric oligonucleotide comprising a phosphorothioate, phosphodiester or phosphorodithioate region and an alkylphosphonate or alkylphosphonothioate region. In certain embodiments, the antisense oligonucleotide comprises a ribonucleotide or 2'-O-substituted ribonucleotide region and a deoxyribonucleotide region.

In a fifth aspect, the invention provides a pharmaceutical composition comprising an inhibitor of a gene comprising an antisense oligonucleotide which inhibits expression the gene in operable association with a protein effector of a product of the gene. In certain embodiments, the composition further comprises a pharmaceutically acceptable carrier.

In a sixth aspect, the invention provides a use for investigating the role of a gene and/or a product of the gene in cellular growth, including the growth of tumor cells. In the use according to this aspect of the invention, the cell type of interest is to be contacted with a synergistic amount of an antisense oligonucleotide which inhibits expression the gene and a synergistic amount of a protein effector of a product of the gene, as described for the first aspect according to the invention, resulting in inhibition of expression of the gene in the cell. In certain embodiments, the gene encodes the product selected from the group consisting of a DNA methyltransferase. The combinations described herein may be administered at different points in the cell cycle, or in conjunction with promoters or inhibitors of cell growth to determine the role of the gene and/or the product of the gene in the growth of the cell type of interest.

The uses, inhibitors, and compositions of the invention that inhibit expression and/or activity of a gene and/or gene product may be used to treat patients having, or predisposed to developing, a disease responsive to inhibition of the gene. For example, an inhibitor or composition of the invention may be administered with a pharmaceutically-acceptable carrier (e.g., physiological sterile saline solution) via any route of administration to patient suffering from a disease responsive to inhibition of a gene in an attempt to alleviate any resulting disease symptoms. For example, an inhibitor or composition of the invention may be used to relieve symptoms of cancer in a patient suffering from cancer, one exemplary, non-limiting disease responsive to inhibition of DNA methyltransferase. Pharmaceutically-acceptable carriers and their formulations are well-known and generally described in, for example, Remington's Pharmaceutical Sciences (18th Edition, ed. A. Gennaro, Mack Publishing Co., Easton, PA, 1990).

### BRIEF DESCRIPTION OF THE DRAWINGS

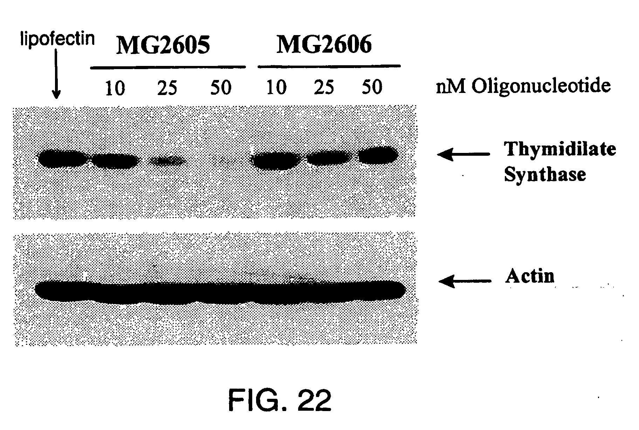

Figures 22-25 are only shown for examplary purposes.

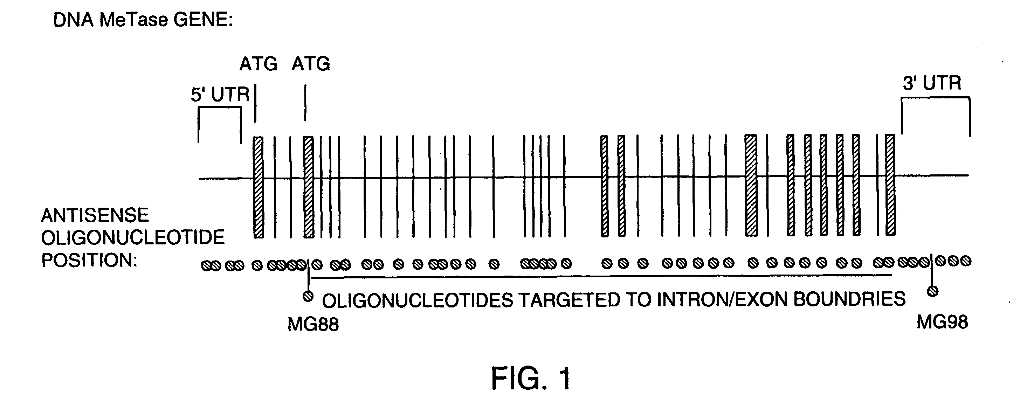

Figure 1 is a diagrammatic representation of the DNA methyltransrerase gene (above) and the positions of the various 20-mer phosphorothioate antisense oligonucleotides (shown as filled circles) on the gene, with the positions of two non-limiting synthetic oligonucleotides, MG88 and MG98, highlighted.

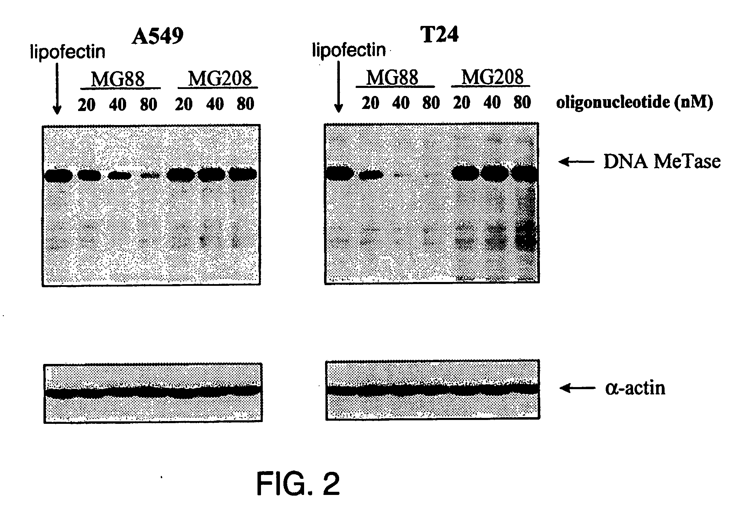

Figure 2 is a representation of autoradiographs of Western blotting analyses showing that treatment with 40 nM or 80 nM of a representative, non-limiting antisense oligonucleotide, MG88, results in an inhibition of MeTase protein expression in both A549 and T24 cells.

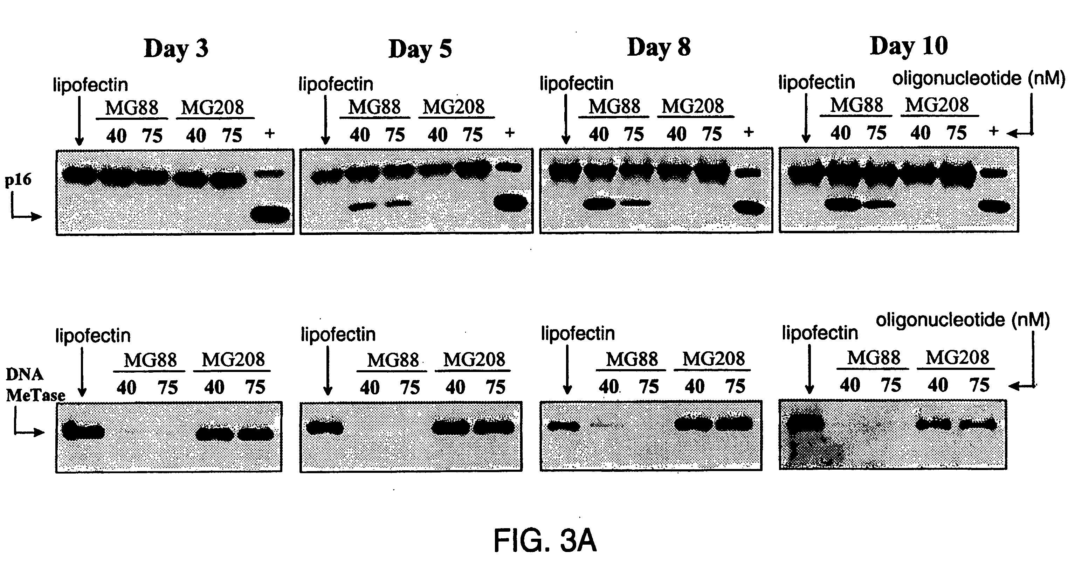

Figure 3A is a representation of autoradiographs of a series of immunoprecipitations followed by Western blotting analyses of T24 cell lysates showing that p16^{ink4} protein levels (upper.panel) increase as DNA MeTase protein levels (lower panel) decrease following treatment of the cells for 3, 5, 8, or 10 days with 40 or 75 nM of a representative, non-limiting antisense oligonucleotide of the invention, MG88, where HeLa cells served as a positive control for p16^{ink4} protein expression.

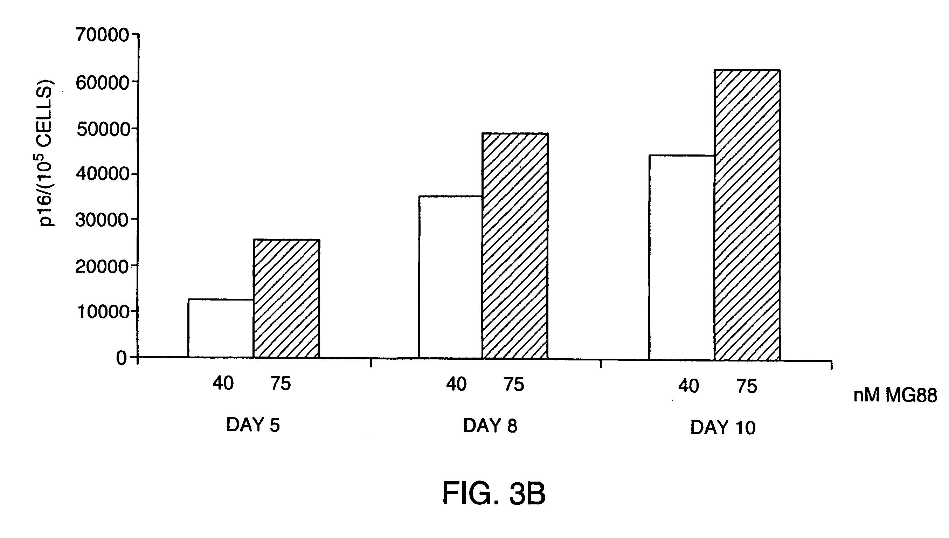

Figure 3B is a graphic representation of p16^{ink4} protein levels normalized to cell number of T24 cells treated with 40 nM or 75 nM MG88 for 3, 5, 8, and 10 days.

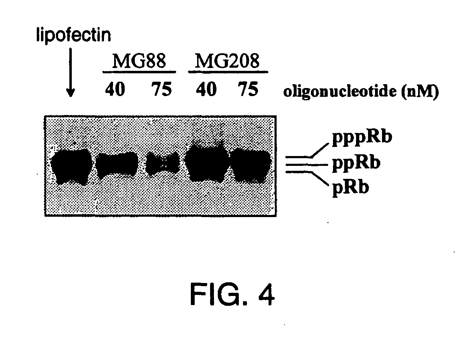

Figure 4 is a representation of an autoradiograph of a Western blotting analysis of T24 cells treated with 40 nM or 75 nM of a representative non-limiting antisense oligonucleotide of the invention, MG88, using an antibody recognizing all phosphorylated forms of Rb.

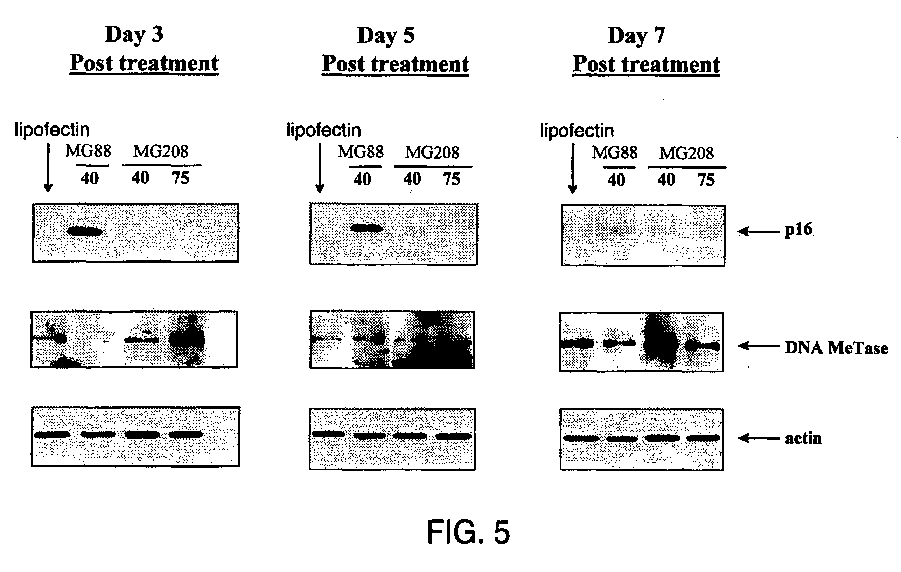

Figure 5 is a representation of autoradiographs of a series of Western blotting analyses of T24 cell lysates prepared 3, 5, or 7 days after cessation of a 10 day treatment of the cells with lipofectin only, 40 nM of MG88, or 40 nM or 75 nM of control oligonucleotide MG208, demonstrating that between 5-7 days post-treatment, DNA MeTase protein expression is restored and p16^{ink4} protein expression is diminished.

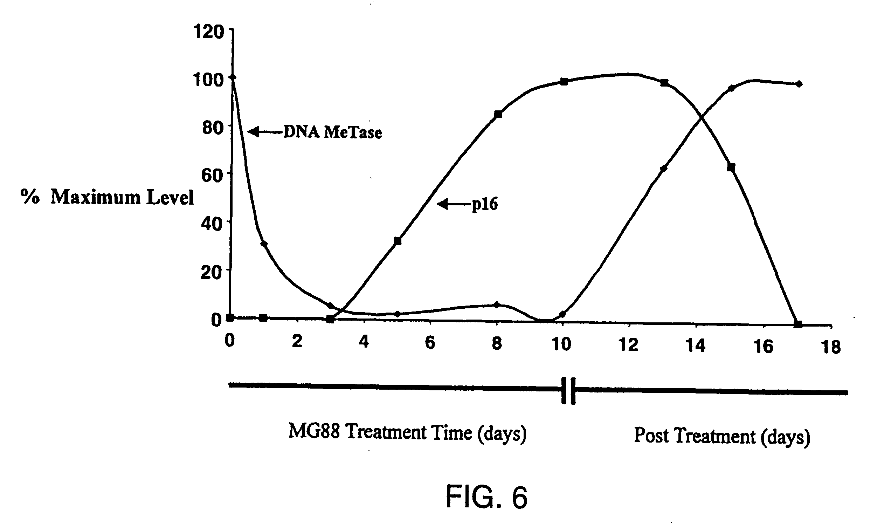

Figure 6 is a graphic representation of the quantitation of DNA MeTase and p16^{ink4} protein levels in T24 cells during ten days of treatment with MG88 and 7 days (*i*.*e*., days 11-17) post-treatment periods. demonstrating the inverse relationship between p16 protein levels and DNA MeTase protein levels in these cells.

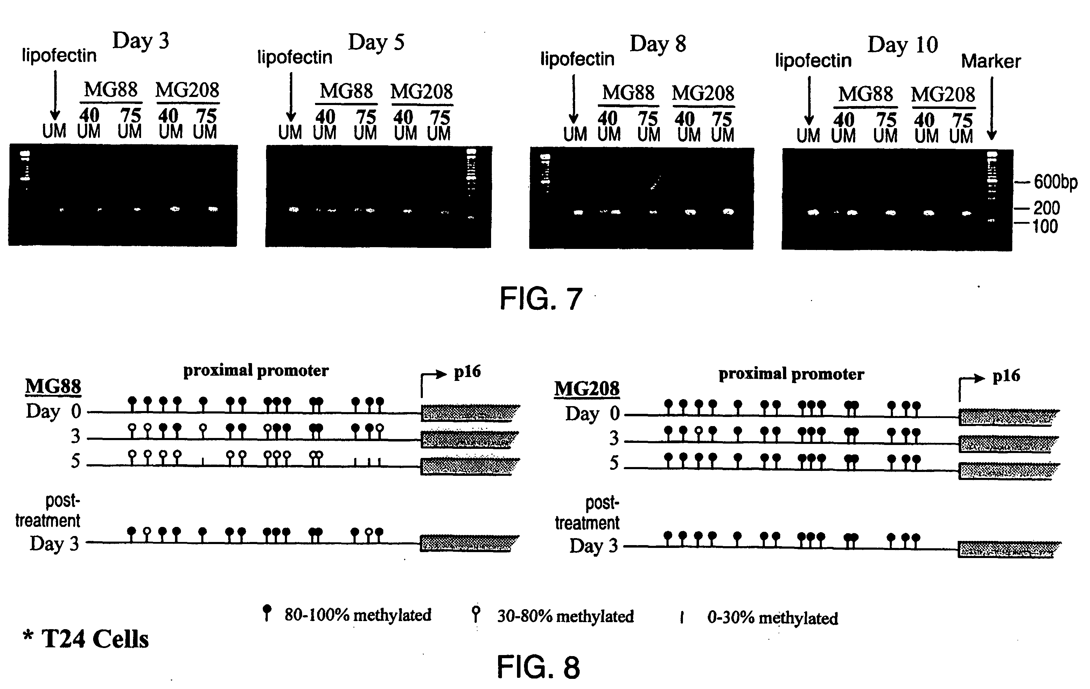

Figure 7 is a representation of a series photographs of showing the methylation-specific PCR (MSP) products resolved on 2% agarose gels of the p16^{ink4} gene promoter from T24 cells treated with 40 or 75 nM of MG88 or MG208 of 3, 5, 8, or 10 days using PCR primers specific for methylated p16^{ink4} (M lanes) or unmethylated p16^{ink4} (U lanes), demonstrating that demethylation of p16^{ink4} occurred only in MG88 treated cells after at least 3 days of treatment.

Figure 8 is a diagrammatic representation of the p16^{ink4} proximal promoter from T24 cells showing the methylation patterns after 0, 3, or 5 days of treatment of the cells with MG88 (left) or control oligonucleotide MG208 (right), demonstrating that reduced methylation occurred only with MG88 treatment. Day 3 post-treatment methylation patterns are shown at the bottom of the figure.

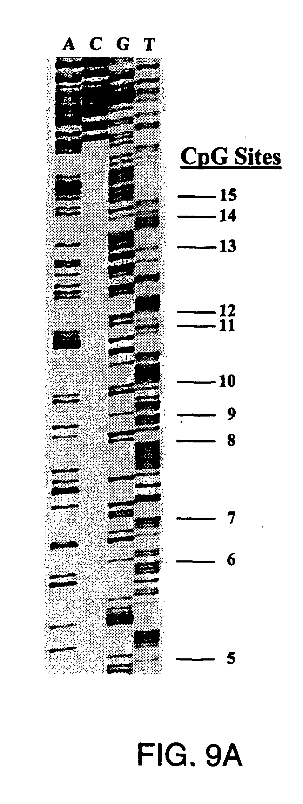

Figure 9A is a representation of a photograph of the results of bisulfite sequencing of the p16^{ink4} gene promoter in p16^{ink4}-expressing clone MG88 C4-5 30 days following cessation of a 5 day treatment with 75 nM MG88, demonstrating that all CpG sites evaluated were not methylated in this clone even after 30 days in culture post-MG88 treatment.

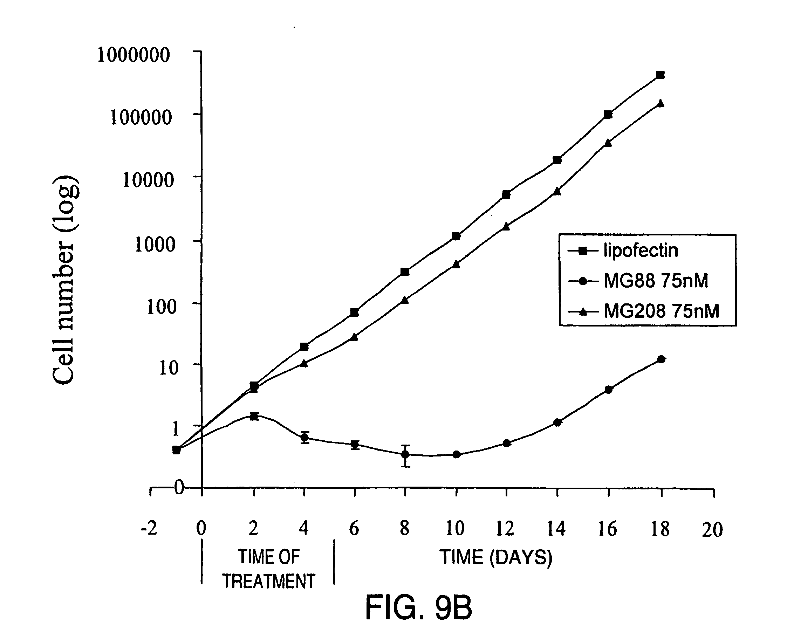

Figure 9B is a graphic representation showing a growth curve of T24 cells during treatment (days 0-5) and post-treatment (days 6-18) with lipofectin only (squares), 75 nM MG88, a non-limiting representative antisense oligonucleotide of the invention (circles), or 75 nM or control oligonucleotide, MG208 (triangles), demonstrating the anti-proliferative effect of MG88.

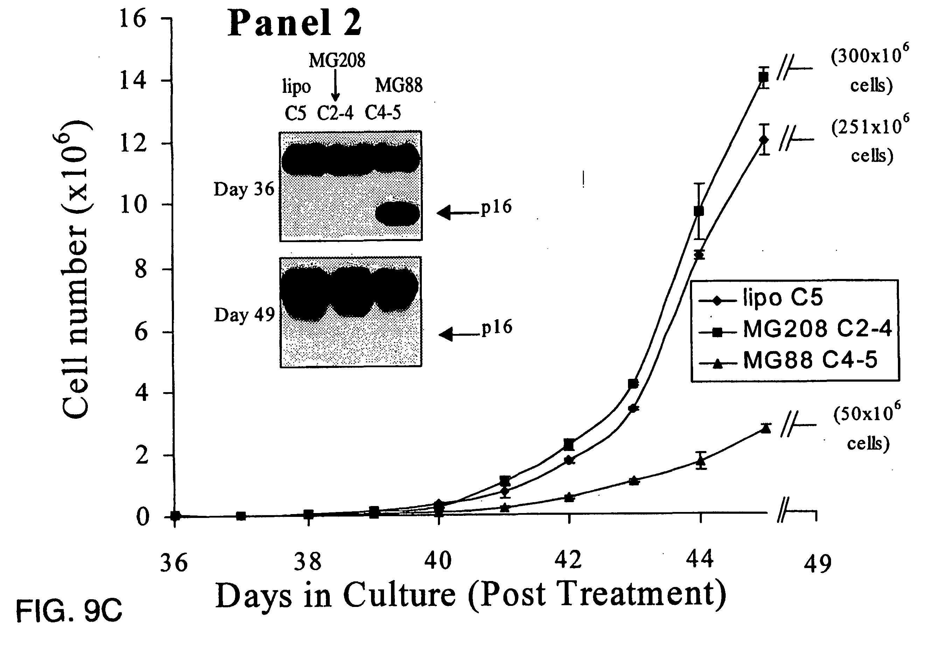

Figure 9C is a graphic representation showing a growth curve during post-treatment of T24 cell clones following a five day treatment with MG88 (clone 4-5, triangles), MG208 (clone 2-4, squares), or lipofectin only (clone 5, diamonds). The inserted representation of two autoradiographs show the p16^{ink4} protein level in each of the clones of days 36 and 49 post-treatment.

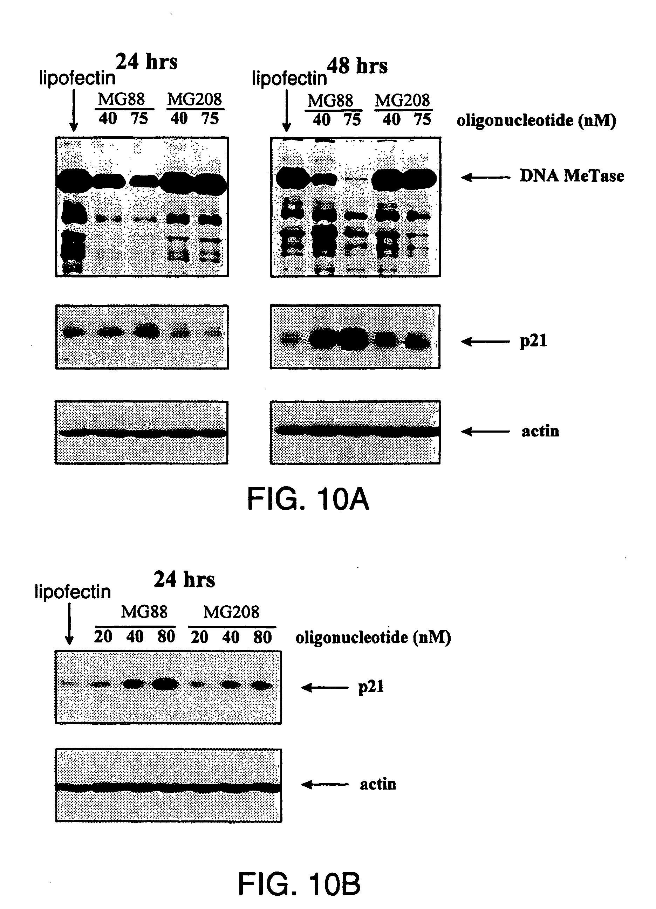

Figure 10A is a representation of autoradiographs of Western blotting analyses of p21^{WAF1}, MeTase, and α-actin protein levels in T24 cells treated for 24 hours (left panels) or 48 hours (right panels) with lipofectin only, or 40 nM or 75 nM of MG88 or MG208, demonstrating that p21^{WAF1} protein expression is induced by inhibition of MeTase expression.

Figure 10B is a representation of autoradiographs of Western blotting analyses showing the dose-response of p21^{WAF1} protein levels in T24 cells treated for 24 hours with 20 nM, 40 nM, or 80 nM of MG88 or MG208. Actin protein levels are shown as a control for protein loading.

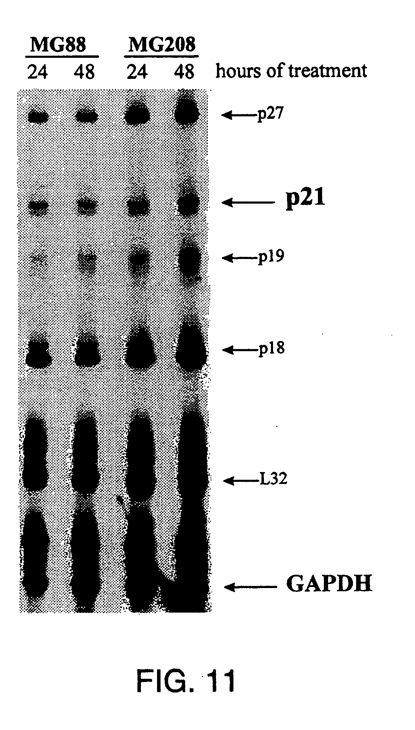

Figure 11 is a representative of an autoradiograph showing a Northern blotting analysis of RNA from T24 cells treated for 24 or 48 hours with MG88 or MG208.

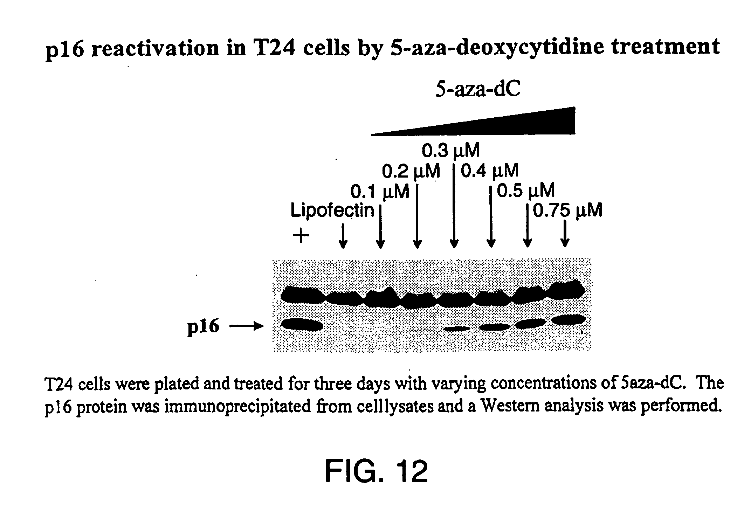

Figure 12 is a representation of an autoradiograph of a Western blotting analysis showing the reactivation of p16 expression in T24 cells following treatment for three days with increasing concentrations of a representative, non-limiting DNA MeTase protein effector, 5-aza-dC.

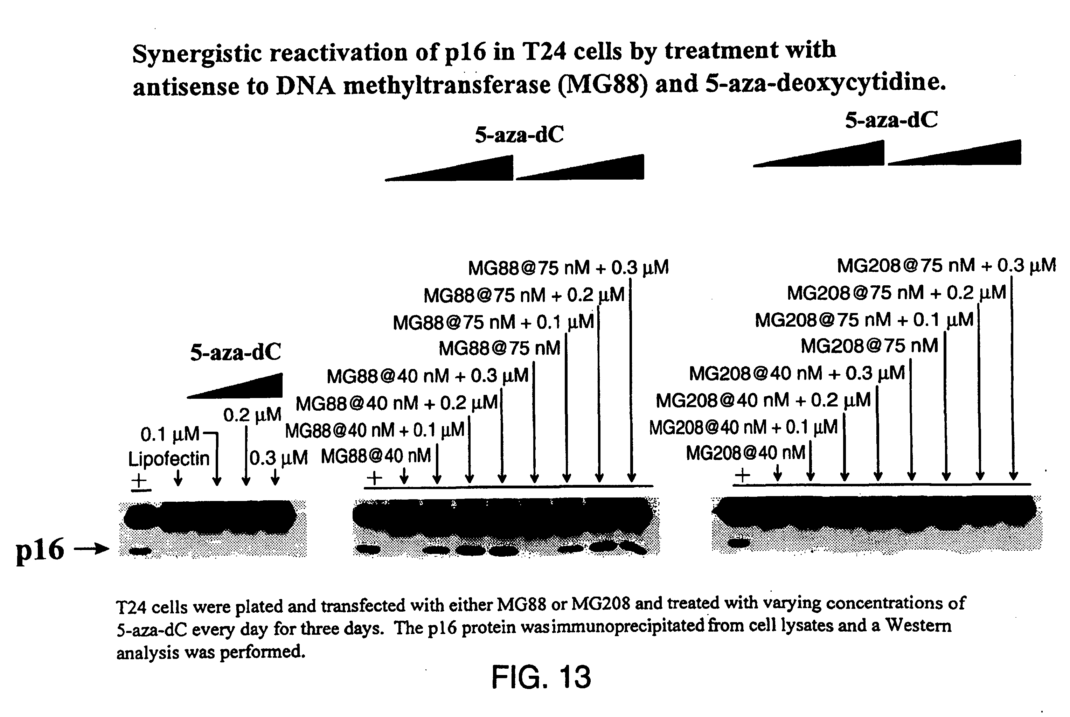

Figure 13 is a representation of an autoradiograph of the Western blot analysis showing the synergistic reactivation of p16 expression in T24 cells following treatment for three days with a representative, nonlimiting, synthetic antisense oligonucleotide (MG88) and/or a representative non-limiting DNA MeTase protein effector (5-aza-dC) according to the invention; the three panels show varying combinations and concentrations of representative antisense oligonucleotides and DNA MeTase protein effectors according to the invention.

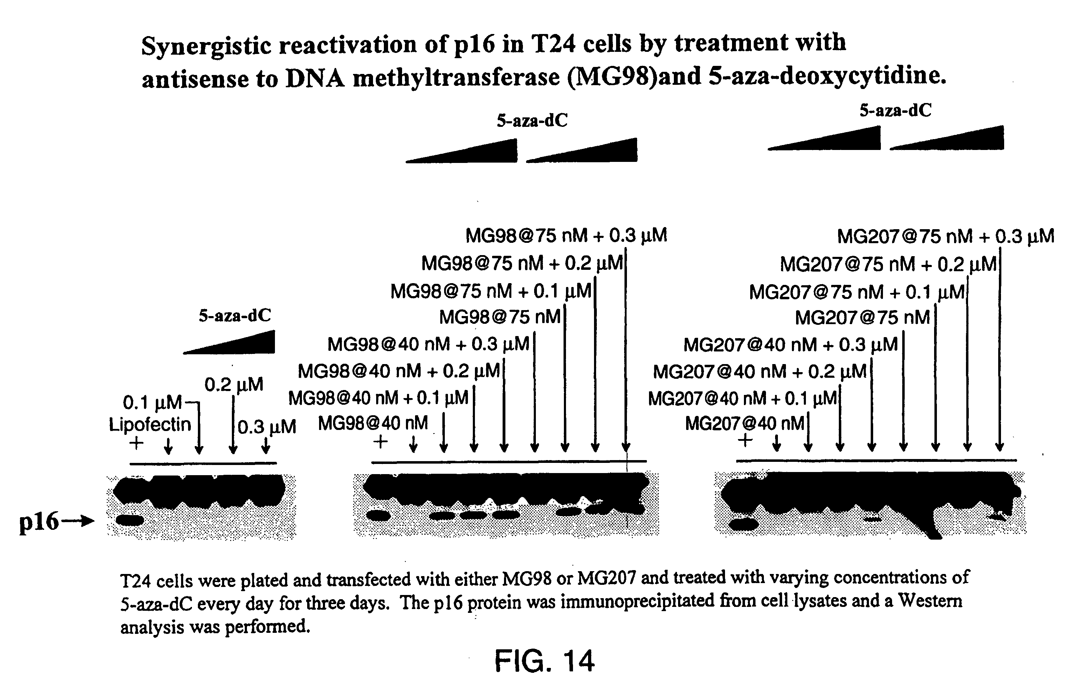

Figure 14 is a representation of an autoradiograph of the Western blot analysis showing the synergistic reactivation of p16 expression in T24 cells following treatment for three days with a representative, nonlimiting, synthetic antisense oligonucleotide (MG98) and/or a representative non-limiting DNA MeTase protein effector (5-aza-dC) according to the invention; the three panels show varying combinations and concentrations of representative oligonucleotides and DNA MeTase protein effectors according to the invention.

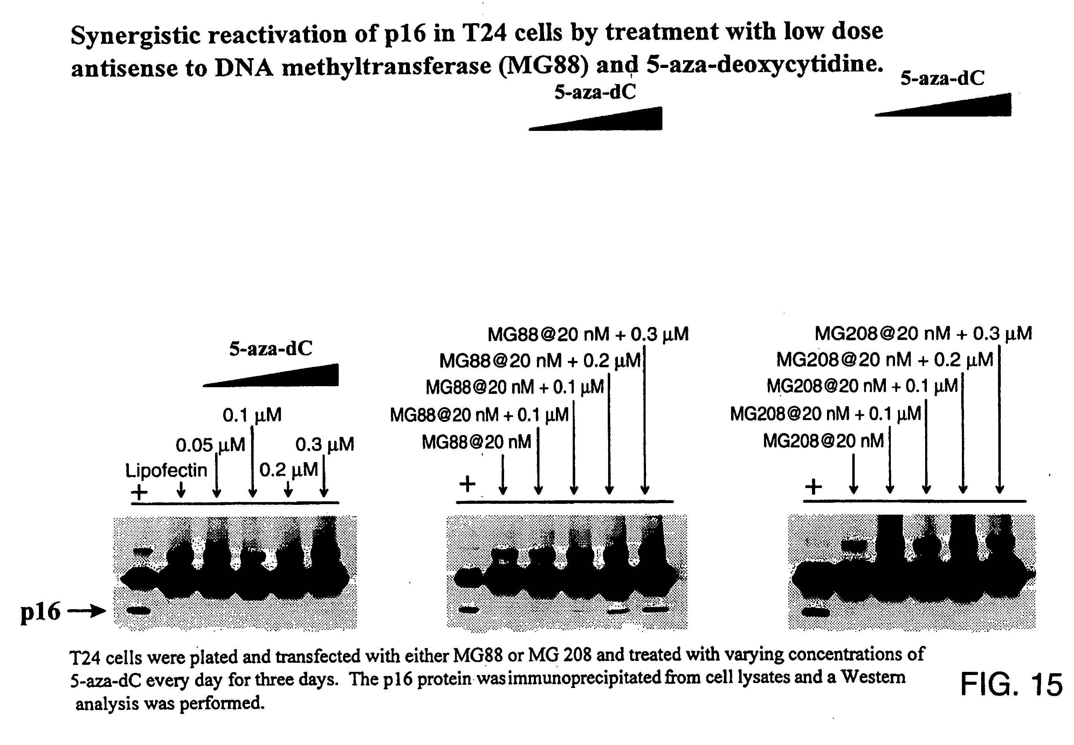

Figure 15 is a representation of an autoradiograph of the Western blot analysis showing the synergistic reactivation of p16 expression in T24 cells following treatment for three days with a representative, nonlimiting, synthetic oligonucleotide (MG88) and/or a representative non-limiting DNA MeTase protein effector (5-aza-dC) according to the invention at low concentrations; the three panels show varying combinations and concentrations of representative oligonucleotides and DNA MeTase protein effectors according to the invention.

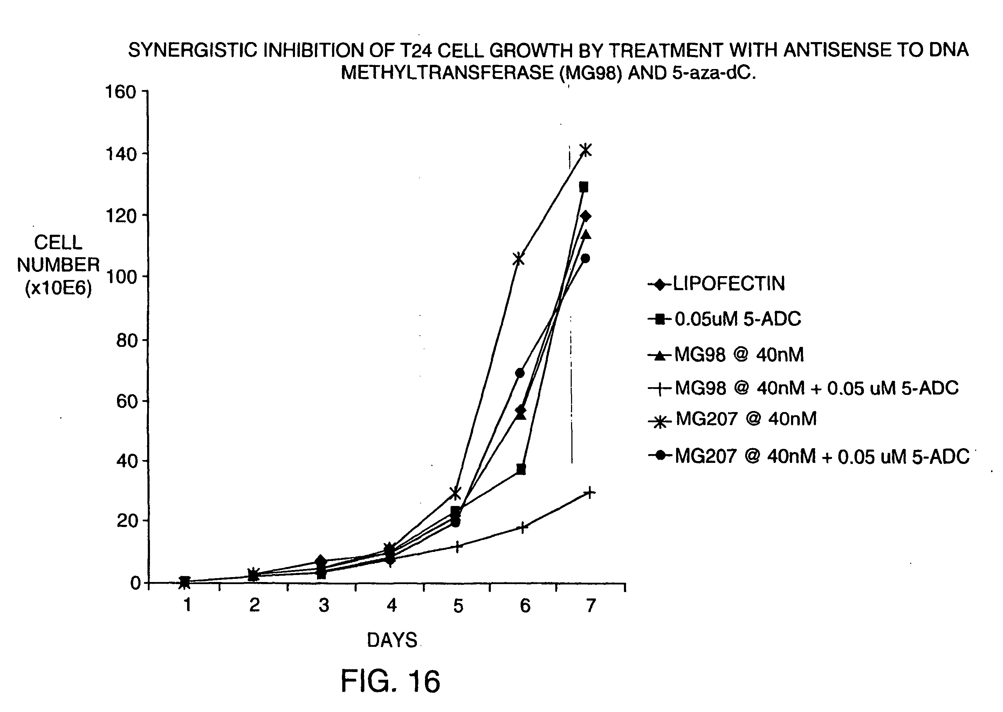

Figure 16 is a graphic representation showing the ability of a representative, nonlimiting, synthetic antisense oligonucleotide (MG98) and of a representative, nonlimiting, DNA MeTase protein effector (5-aza-dC) according to the invention to inhibit T24 human bladder cancer cell growth in a synergistic fashion resulting in an increased inhibitory effect as compared to that observed using either only the antisense oligonucleotides or only the DNA MeTase protein effectors.

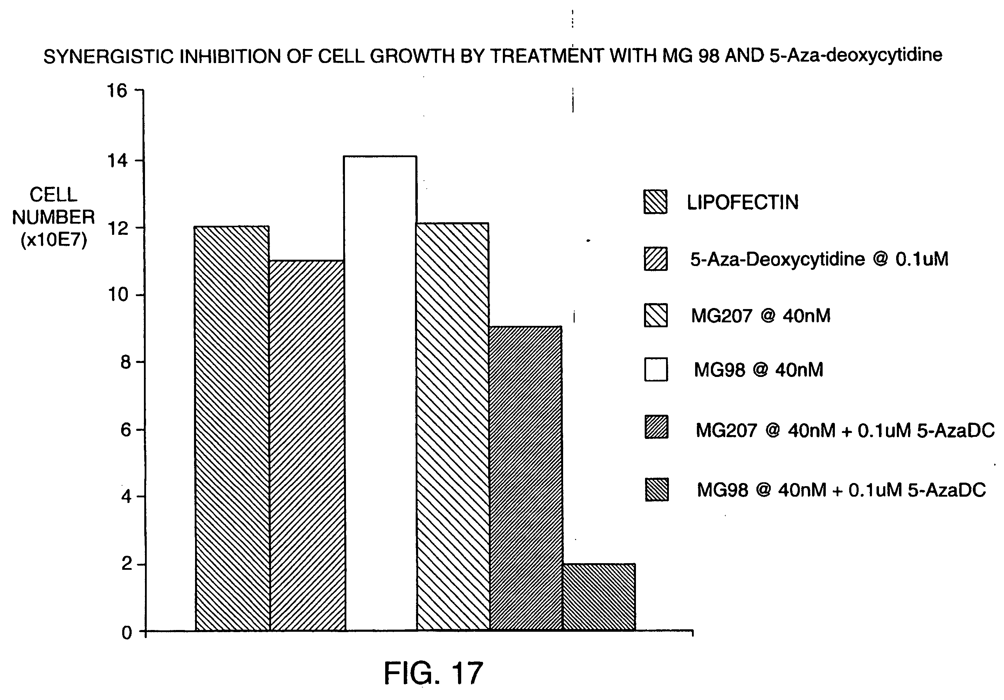

Figure 17 is a graphic representation showing the synergistic inhibition of T24 human bladder cancer cell growth after treatment for seven days with lipofectin only (first bar from the left); 1 µM of a representative, nonlimiting, DNA MeTase protein effector, 5-aza-dC (second bar from the left); 40 nM of control synthetic oligonucleotide MG207 (third bar from the left); 40 nM of a representative nonlimiting synthetic MeTase antisense oligonucleotide, MG98 (fourth bar from the left); MG207 plus 5-aza-dC (fifth bar from the left); or MG98 plus 5-aza-dC (sixth bar from the left).

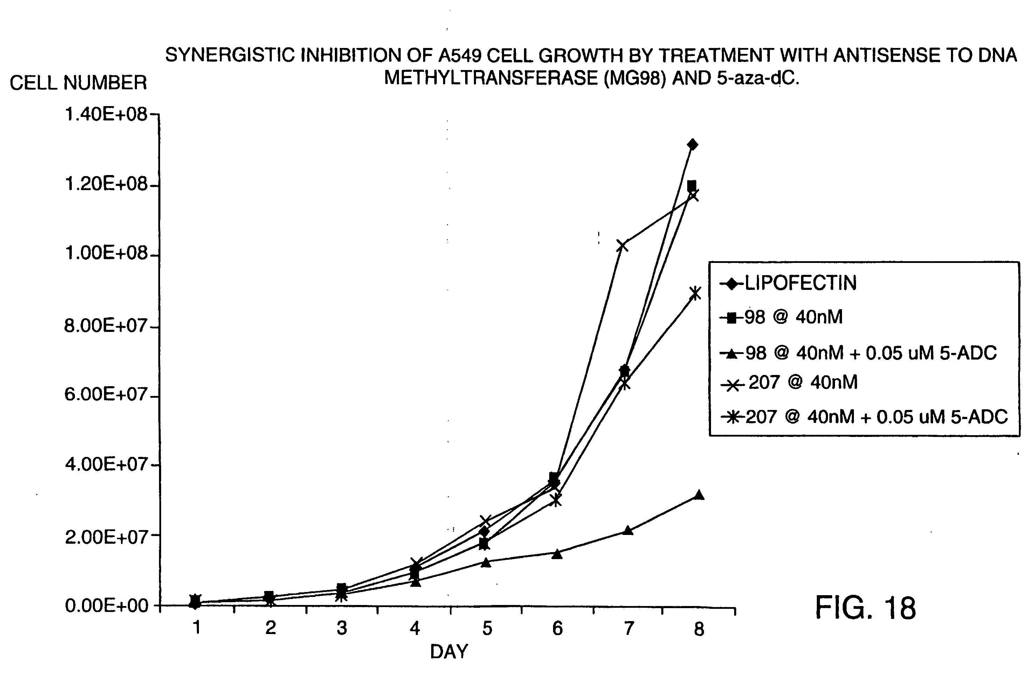

Figure 18 is a graphic representation showing the ability of a representative, nonlimiting, synthetic oligonucleotide (MG98) and of a representative, nonlimiting, DNA MeTase protein effector (5-aza-dC) according to the invention to inhibit A549 human non-small cell lung cancer cell growth in a synergistic fashion resulting in an increased inhibitory effect as compared to that observed using either only the oligonucleotides or only the DNA MeTase protein effectors.

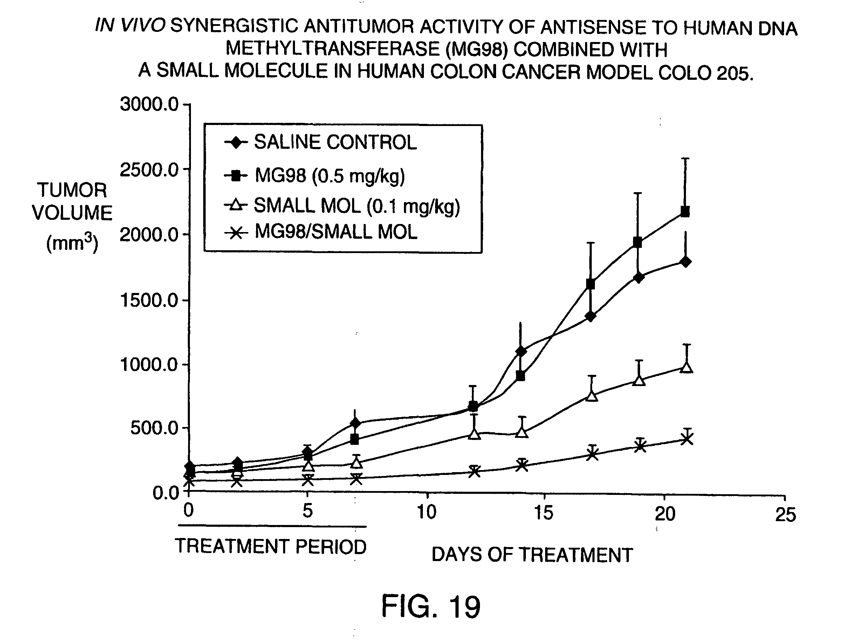

Figure 19 is a graphic representation showing the ability of a representative, nonlimiting, synthetic oligonucleotide (MG98) and of a representative, nonlimiting, DNA MeTase protein effector (5-aza-dC) according to the invention to inhibit Colo 205 human colon cancer cell growth (expressed as tumor volume over time) in a synergistic fashion resulting in an increased inhibitory effect as compared to that observed using either only the oligonucleotides or only the DNA MeTase protein effectors.

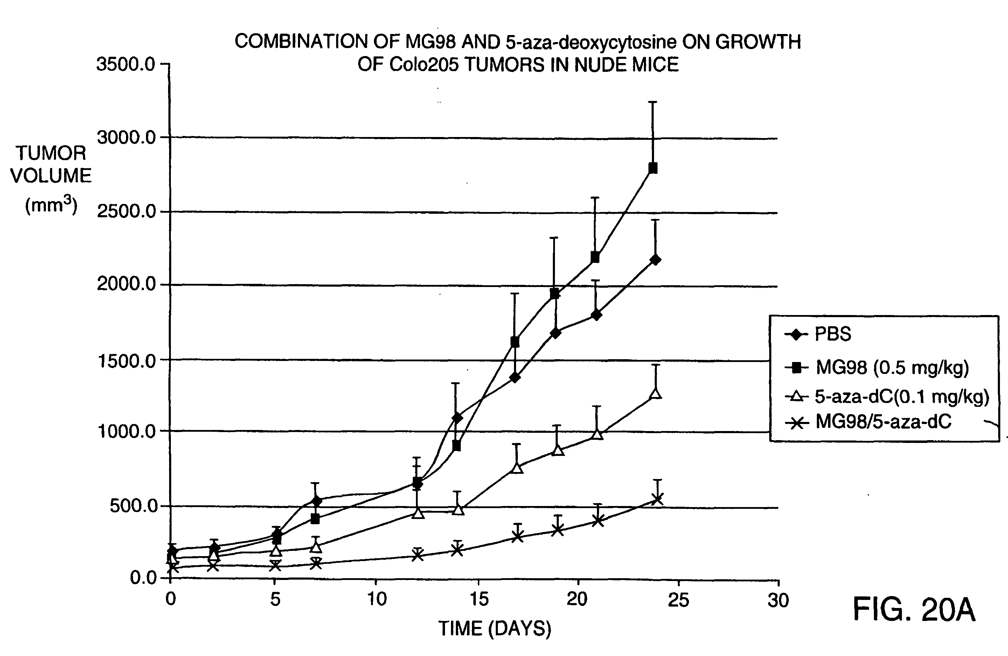

Figure 20A is a graphic representation showing the inhibition of Colo 205 tumor cell growth in nude mice following treatment of the mice with saline (diamond); 0.5 mg/kg of a representative, nonlimiting MeTase synthetic oligonucleotide, MG98 (square); 0.1 mg/kg of a representative, nonlimiting, DNA MeTase protein effector, 5-aza-dC (triangle); or a combination of both MG98 plus 5-aza-dC (X).

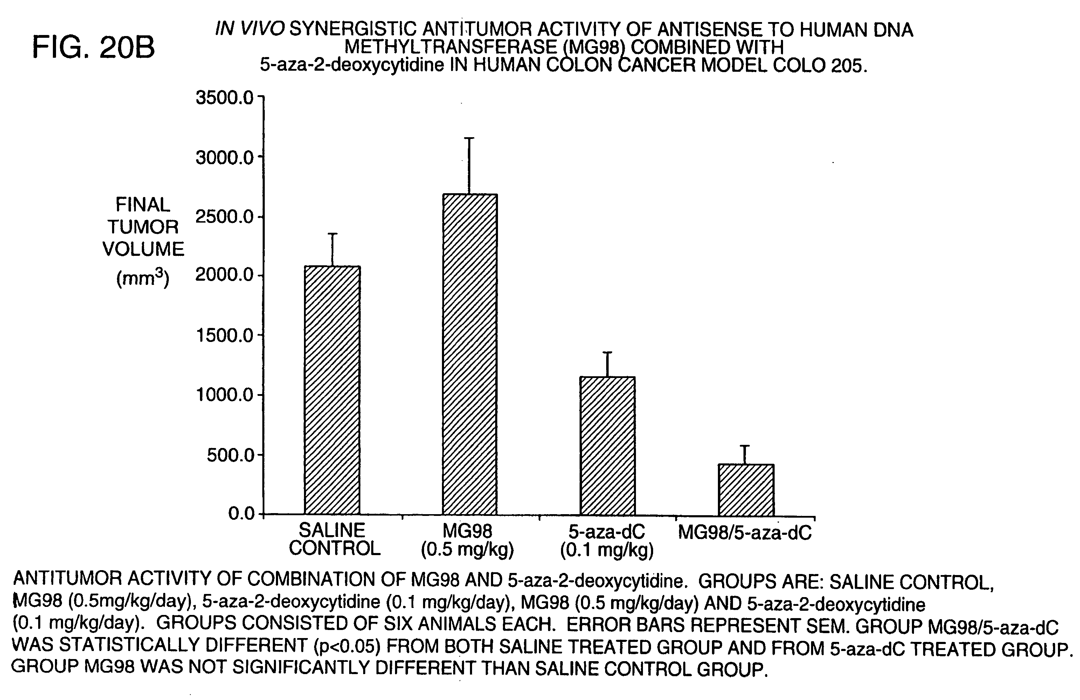

Figure 20B is a graphic representation showing the inhibition of Colo 205 human colon cancer cell growth (expressed as final tumor volume) by a representative, nonlimiting, synthetic oligonucleotide (MG98) and by a representative, nonlimiting DNA MeTase protein effector (5-aza-dC) according to the invention, in a synergistic fashion, resulting in an statistically increased inhibitory effect (p<0.05) as compared to that observed using either only oligonucleotide, protein effector, or saline.

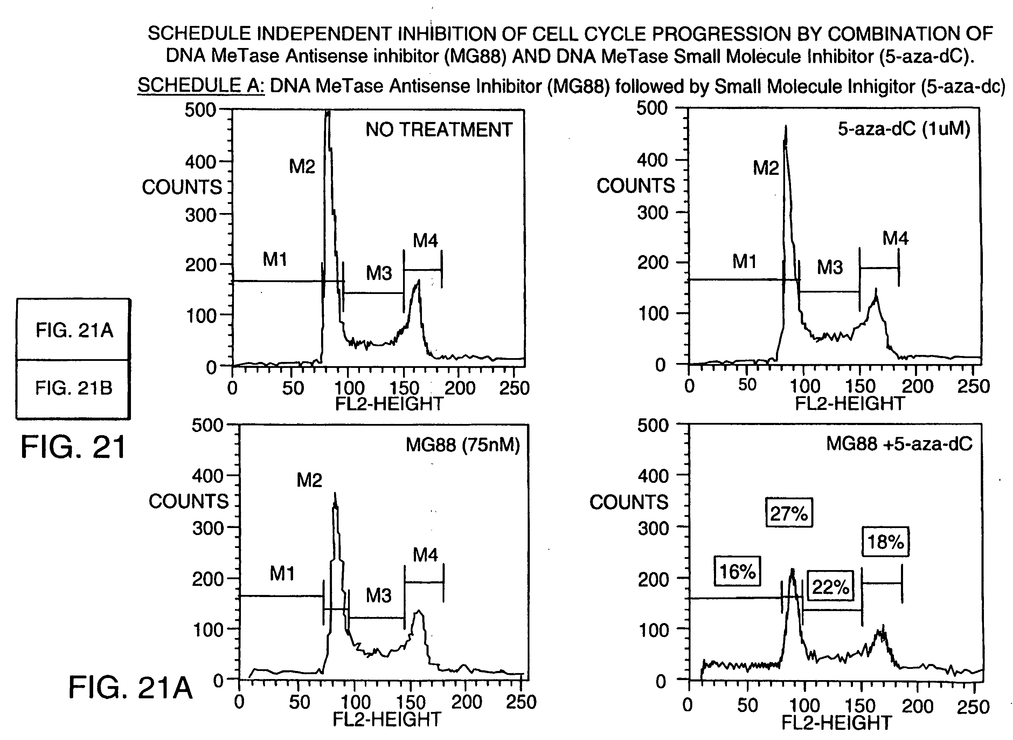

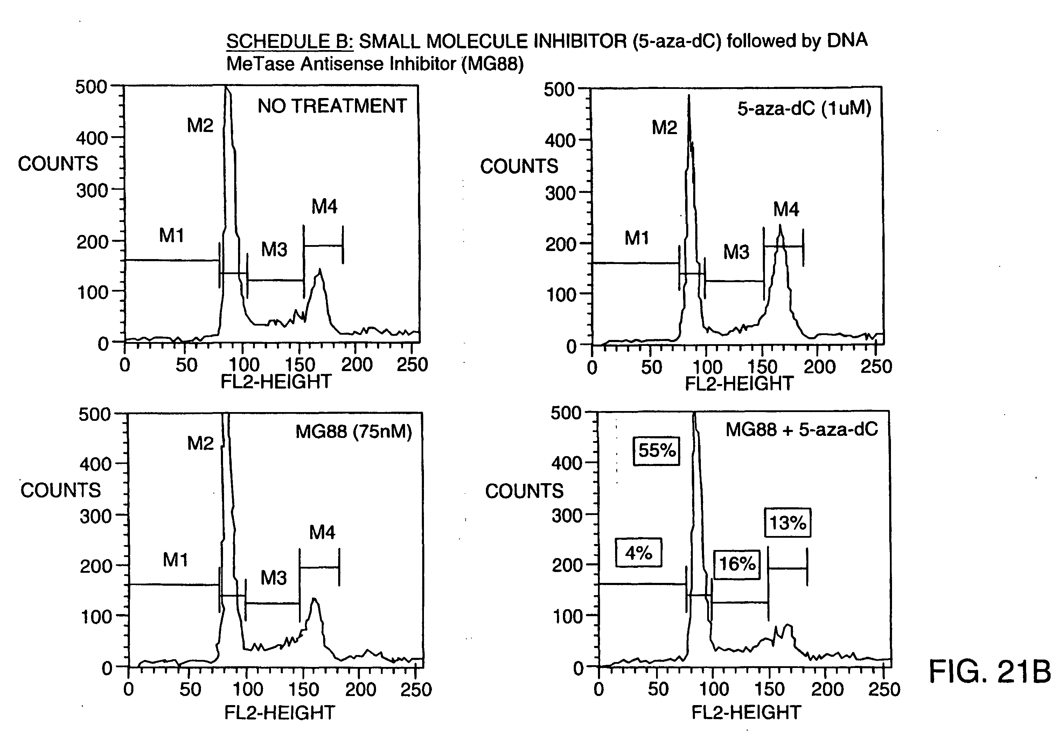

Figure 21 is a schematic diagram showing a series of FACS histogram analyses of T24 cells treated with a representative, nonlimiting, synthetic oligonucleotide (MG88) and/or a representative non-limiting DNA MeTase protein effector (5-aza-dC) according to the invention, at different schedules. The upper panel of histograms shows cells treated on schedule A, where MG88 is administered before 5-aza-dC. The lower panel of histograms shows cells treated on schedule B, where 5-aza-dC is administered before MG88.

Figure 22 is a representation of an autoradiograph of the Western blotting analysis showing the synergistic inhibition of thymidylate synthase protein expression in T24 cells using the combination of a representative, nonlimiting, synthetic antisense oligonucleotide (MG2605) and a representative, nonlimiting TS protein effector (5-FU) according to the invention.

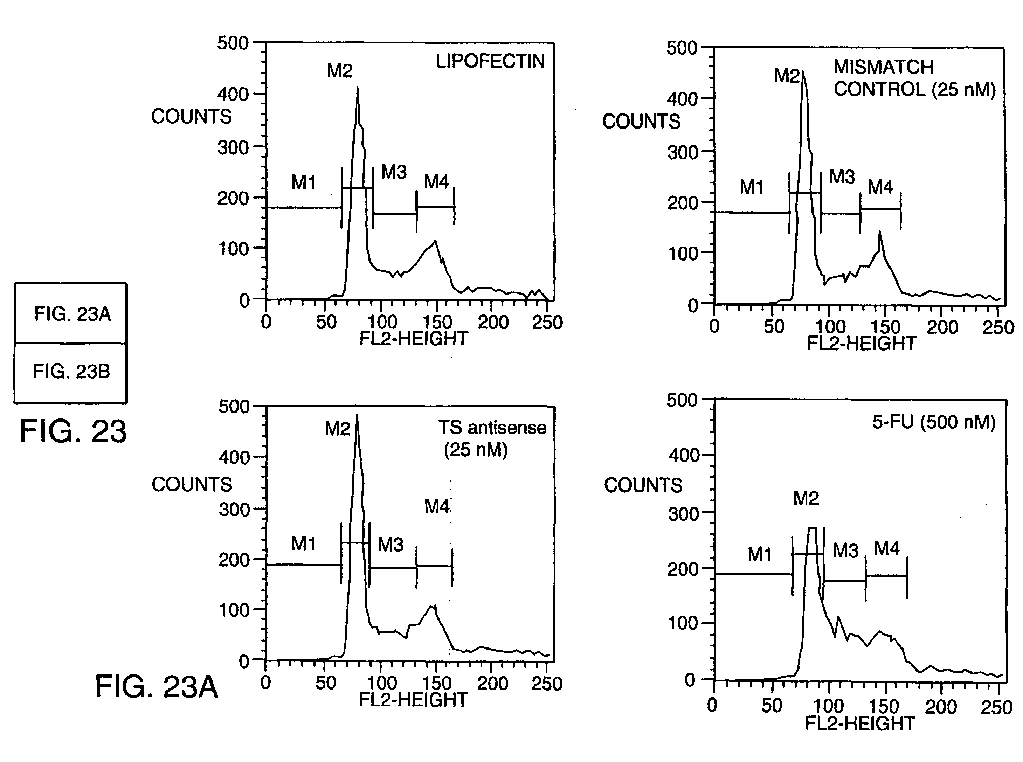

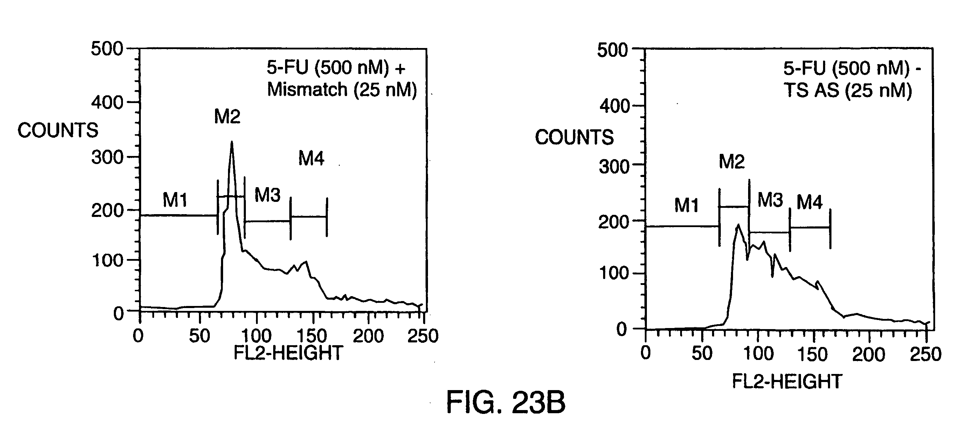

Figure 23 is a schematic diagram showing a series of FACS histogram analyses of T24 cells treated with a representative, nonlimiting synthetic thymidylate synthase antisense oligonucleotide and/or a representative non-limiting TS protein effector (5-FU) according to the invention. The top histograms shows cells treated with lipofectin only; the second histogram shows cells treated with 25 nM mismatch control oligonucleotide; the third histogram from the top shows cells treated with 25 nM of the TS antisense oligonucleotide, MG2605; the fourth histogram from the top shows cells treated with 500 nM of 5-FU; the fifth histogram from the top shows cells treated with 5-FU plus mismatch oligonucleotide; and the sixth histogram (i.e., the bottom histogram) shows cells treated with 5-FU plus the TS antisense oligonucleotide, MG2605.

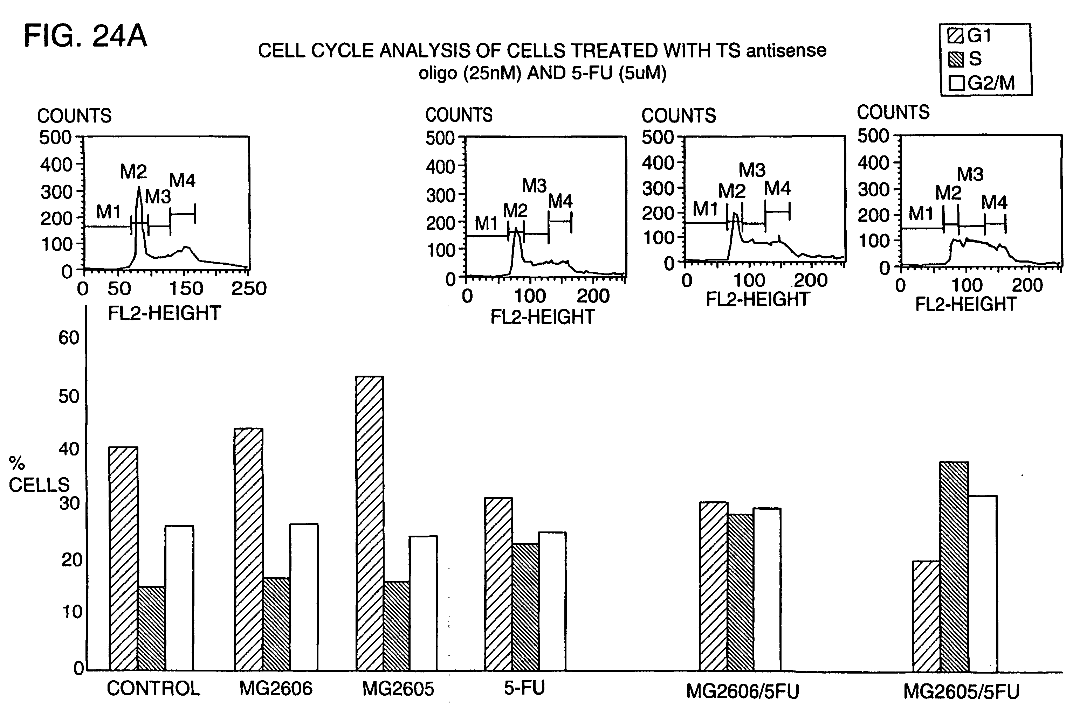

Figure 24A is a graphic representation showing the percentages of T24 cells in the G₁ phase (gray bars; section M2 on inserted histograms) S phase (black bars; section M3 on inserted histograms), and G₂/M phase (white bars; section M4 on inserted histogram) following treatment with 25 nM of a representative, nonlimiting TS antisense oligonucleotide MG2605, 25 nM of control oligonucleotide MG2606, 5 µM of a representative nonlimiting TS protein effector, 5-FU, or a combination of oligonucleotide plus 5-FU.

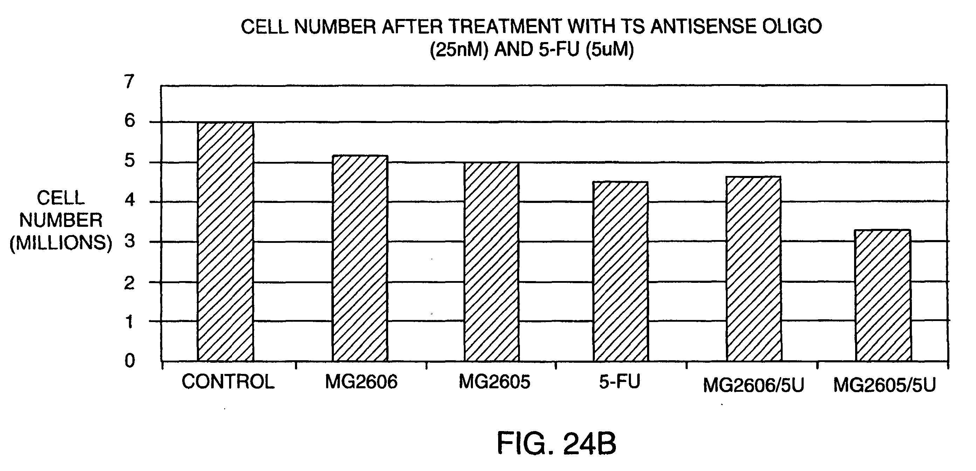

Figure 24B is a graphic representation of the number of T24 cells remaining following treatment with following no treatment; or treatment with 25 nM of a representative, nonlimiting TS antisense oligonucleotide MG2605; 25 nM of control oligonucleotide MG2606; 5 µM of a representative nonlimiting TS protein effector, 5-FU; or a combination of MG2605 or MG2606 plus 5-FU.

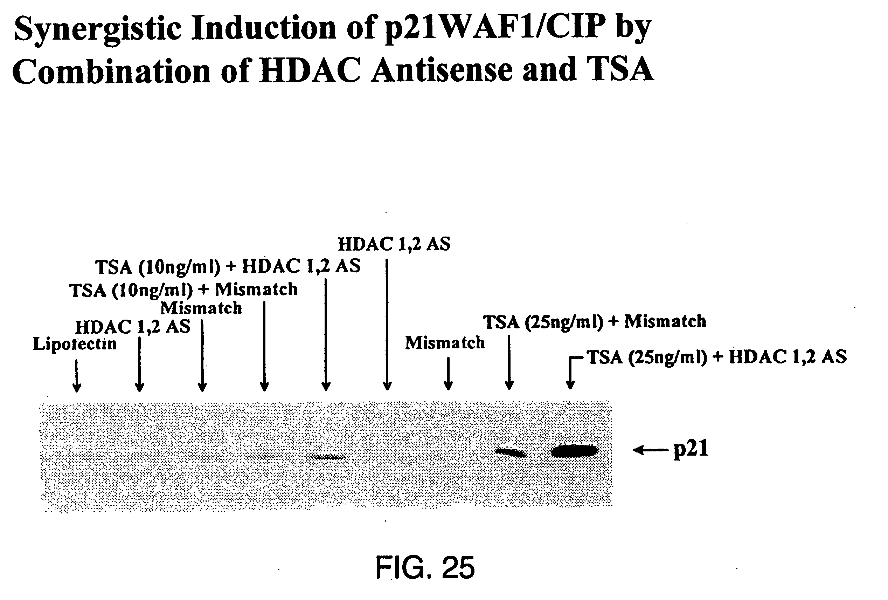

Figure 25 is a representation of an autoradiograph of the Western blotting analysis showing the synergistic induction of p21^{WAF1} by the combination of a representative, nonlimiting, synthetic HDAC antisense oligonucleotide and a representative, nonlimiting HDAC protein effector (TSA) according to the invention.

### DETAILED DESCRIPTION OF THE PREFERRED EMBODIMENTS

The present inventors have devised a combination approach by which expression of a gene is inhibited at the genetic level as well as at the protein level. Surprisingly, this combination approach has been found to reduce the required dosage for efficacy of both antisense oligonucleotides against the gene itself as well as of protein effectors against the product of the gene.

The invention provides improved uses, compounds (*e*.*g*., inhibitors such as antisense oligonucleotides and protein effectors) and compositions for the modulation of gene expression at the genetic level as well as at the protein level. In addition, where the gene is DNA methyltransferase (DNA MeTase), the invention provides uses, compounds, and compositions for the modulation of mammalian gene expression regulated by methylation. The uses and compositions according to the invention are useful as analytical tools for transgenic studies and as therapeutic tools, including as gene therapy tools. The invention also provides uses and compositions which may be manipulated and fine tuned to fit the condition(s) to be treated while producing fewer side effects. Standard reference works setting forth the general principles of recombinant DNA technology include Ausubel *et al.,* Current Protocols in Molecular Biology, John Wiley & Sons, New York (1994); Sambrook *et al*., Molecular Cloning: A Laboratory Manual, 2d Ed., Cold Spring Harbor Laboratory Press, Plainview, New York (1989); Kaufman *et al*., Eds., Handbook of Molecular and Cellular Methods in Biology and Medicine, CRC Press, Boca Raton (1995); McPherson, Ed., Directed Mutagenesis: A Practical Approach, IRL Press, Oxford (1991). The patents and scientific literature, including accession numbers to GenBank database sequences, referred to herein establish the knowledge of those with skill in the art. Any conflict between any reference cited herein and the specific teachings of this specification shall be resolved in favor of the later. Likewise, any conflict between an art-understood definition of a word or phrase and a definition of the word or phrase as specifically taught in this specification shall be resolved in favor of the latter.

In a first aspect the invention provides a use of an effective synergistic amount of an antisense oligonucleotide which inhibits expression of a gene, and an effective synergistic amount of a protein effector of a product of the gene for the preparation of a pharmaceutical composition for inhibiting the expression of a gene in a cell, wherein the cell is to be contacted with said effective synergistic amounts. In certain embodiments of the first aspect of the invention, the cell is to be contacted with an effective synergistic amount of at least one antisense oligonucleotide and/or at least one protein effector for an effective period of time.

As used herein for all aspects of the invention, the term "protein effector" denotes an active moiety capable of inhibiting the indicated protein or product of a gene at the protein level. For example, a "DNA MeTase protein effector" or a "protein effector of the product of a DNA MeTase gene" inhibits DNA MeTase at the protein level. By "product of a gene" is meant a protein or polypeptide, regardless of secondary modifications (*e.g.*, glycosylation, lipidation, or phosphorylation), encoded by the gene. The term protein effector therefore includes, without limitation, specific enzyme inhibitors which are capable of inhibiting activity of the indicated protein or product of a gene. A protein effector is a molecule that inhibits the activity of the indicated protein to a greater extent than it inhibits the activity of any unrelated protein. Preferably, a protein effector inhibits the indicated protein at least 5-fold, more preferably at least 10-fold, even more preferably at least 50-fold, and most preferably at least 100-fold more than it inhibits any unrelated protein.

The terms "effective synergistic amount" and "effective period of time" are used to denote known concentrations of the antisense oligonucleotide and of the protein effector and for periods of time effective to achieve the result sought. The effective synergistic amount of the antisense oligonucleotide and/or the effective synergistic amount of the protein effector is/are less than the amount(s) empirically found necessary to inhibit the gene when either the antisense oligonucleotide or the protein effector are used individually. In preferred embodiments, the combined inhibitory effect of the antisense oligonucleotide and the protein effector according to the invention are more than additive, *i*.*e*., the combined inhibitory effect is greater than the expected total inhibitory effect calculated on the basis of the sum of the individual inhibitory effects.

The antisense oligonucleotides according to all aspects of the invention are complementary to a region of double-stranded DNA or of RNA (or a region at the intron/exon boundary of DNA or RNA) that encodes the product of the gene. For purposes of the invention, the term "oligonucleotide" includes polymers of two or more deoxyribonucleosides, ribonucleosides, or 2'-O-substituted ribonucleoside residues, or any combination thereof. Preferably, such oligonucleotides have from about 6 to about 100 nucleoside residues, more preferably from about 8 to about 50 nucleoside residues, and most preferably from about 12 to about 30 nucleoside residues. The nucleoside residues may be coupled to each other by any of the numerous known internucleoside linkages. Such internucleoside linkages include without limitation phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleotide linkages. In certain preferred embodiments, these internucleoside linkages may be phosphodiester, phosphotriester, phosphorothioate, or phosphoramidate linkages, or combinations thereof. The term oligonucleotide also encompasses such polymers having chemically modified bases or sugars and/ or having additional substituents, including without limitation lipophilic groups, intercalating agents, diamines and adamantane. For purposes of the invention the term "2'-O-substituted" means substitution of the 2' position of the pentose moiety with an -0-lower alkyl group containing 1-6 saturated or unsaturated carbon atoms, or with an -0-aryl or allyl group having 2-6 carbon atoms, wherein such alkyl, aryl or allyl group may be unsubstituted or may be substituted, e.g., with halo, hydroxy, trifluoromethyl, cyano, nitro, acyl, acyloxy, alkoxy, carboxyl, carbalkoxyl, or amino groups; or such 2' substitution may be with a hydroxy group (to produce a ribonucleoside), an amino or a halo group, but not with a 2'-H group.

Particularly preferred antisense oligonucleotides utilized in this aspect of the invention include chimeric oligonucleotides and hybrid oligonucleotides.

For purposes of the invention, a "chimeric oligonucleotide" refers to an oligonucleotide having more than one type of internucleoside linkage. One preferred example of such a chimeric oligonucleotide is a chimeric oligonucleotide comprising a phosphorothioate, phosphodiester or phosphorodithioate region, preferably comprising from about 2 to about 12 nucleotides, and an alkylphosphonate or alkylphosphonothioate region (see *e*.*g*., Pederson *et al*. U.S. Patent Nos. 5,635,377 and 5,366,878). Preferably, such chimeric oligonucleotides contain at least three consecutive internucleoside linkages selected from phosphodiester and phosphorothioate linkages, or combinations thereof.

For purposes of the invention, a "hybrid oligonucleotide" refers to an oligonucleotide having more than one type of nucleoside. One preferred example of such a hybrid oligonucleotide comprises a ribonucleotide or 2'-O-substituted ribonucleotide region, preferably comprising from about 2 to about 12 2'-O-substituted nucleotides, and a deoxyribonucleotide region. Preferably, such a hybrid oligonucleotide will contain at least three consecutive deoxyribonucleosides and will also contain ribonucleosides, 2'-O-substituted ribonucleosides, or combinations thereof (see *e.g.*, Metelev and Agrawal, U.S. Patent No. 5,652,355).

The exact nucleotide sequence and chemical structure of an antisense oligonucleotide utilized in the invention can be varied, so long as the oligonucleotide retains its ability to inhibit expression of the gene of interest. This is readily determined by testing whether the particular antisense oligonucleotide is active by quantitating the mRNA encoding a product of the gene, or in a Western blotting analysis assay for the product of the gene, or in an activity assay for an enzymatically active gene product, or in a soft agar growth assay, or in a reporter gene construct assay, or an *in vivo* tumor growth assay, all of which are described in detail in this specification or in Ramchandani *et al*. (1997) Proc. Natl. Acad. Sci. USA 94: 684-689.

Antisense oligonucleotides utilized in the invention may conveniently be synthesized on a suitable solid support using well known chemical approaches, including H-phosphonate chemistry, phosphoramidite chemistry, or a combination of H-phosphonate chemistry and phosphoramidite chemistry (*i*.*e*., H-phosphonate chemistry for some cycles and phosphoramidite chemistry for other cycles). Suitable solid supports include any of the standard solid supports used for solid phase oligonucleotide synthesis, such as controlled-pore glass (CPG) (see, e.g., Pon, R.T. (1993) Methods in Molec. Biol. 20: 465-496).

In certain embodiments of all aspects of the invention, any of the antisense oligonucleotides may be operably associated with one or more protein effectors. A preferred operable linkage is a hydrolyzable association. Preferably, the hydrolyzable association is a covalent linkage between the antisense oligonucleotide and the protein effector(s). Preferably, such covalent bonding is hydrolyzable by esterases and/or amidases. Examples of such hydrolyzable bonding are shown in PCT publication WO96/07392, which is hereby incorporated by reference. Phosphate esters are particularly preferred.

In certain preferred embodiments, the covalent linkage may be directly between the antisense oligonucleotide and the protein effector so as to integrate the protein effector into the backbone. Alternatively, the covalent linkage may be through an extended structure. Linkages of this type may be formed by covalently linking the antisense oligonucleotide to the protein effector through coupling of both the antisense oligonucleotide and the protein effector to a carrier molecule such as a carbohydrate, a peptide or a lipid or a glycolipid. Other preferred operable linkages include lipophilic association, such as formation of a liposome containing oligonucleotide and the protein effector covalently linked to a lipophilic molecule and thus associated with the liposome. Such lipophilic molecules include without limitation phosphotidylcholine, cholesterol and phosphatidylethanolamine, and synthetic neoglycolipids, such as syalyllacNAc-HDPE. In certain preferred embodiments, the operable association may not be a physical association, but simply a simultaneous existence in the body, for example, when the antisense oligonucleotide is associated with one liposome and the protein effector is associated with another liposome.

The use and compositions according to the invention are useful for a variety of purposes. For example, they can be used as "probes" of the physiological function of a gene product, wherein they are to be used to inhibit the activity and/or expression of the gene product in an experimental cell culture or animal system and to evaluate the effect of inhibiting such activity and/or expression. This is accomplished, wherein an antisense oligonucleotide which inhibits expression of a gene and a protein effector of a product of the gene according to the invention is to be administered to a cell or an animal and observing any phenotypic effects. This use according to the invention is preferable to traditional "gene knockout" approaches because it is easier to use, and can be used to inhibit the gene and/or a product of the gene at selected stages of development or differentiation. For example, where the gene encodes DNA MeTase, the use according to the invention can serve as a probe to test the role of DNA methylation in various stages of development.

Finally, the uses and compositions according to the invention are useful in therapeutic approaches to human diseases including benign and malignant tumors involving the modulation and/or the suppression of gene expression. The anti-tumor utility of antisense oligonucleotides according to the invention is described in detail elsewhere in this specification.

All of the aspects of the invention disclosed herein are applicable to the synergistic inhibition of any target gene.

More specifically, the invention relates to the inhibition of any target gene by the concurrent or sequential inhibition of the same target gene at both the genetic level (i.e., at either the DNA or the mRNA level) and at the protein level. As exemplified herein for DNA MeTase, such uses and compositions are useful for a variety of purposes. The invention results in an improved inhibitory effect, thereby reducing the effective concentrations of either or both the gene level and the protein levels inhibitors required to obtain a given inhibitory effect as compared to those necessary when either inhibitor is used individually.

Thus, the uses and compositions according to all aspects of the invention are useful in therapeutic approaches to human diseases involving the modulation and/or suppression of gene expression of a particular target gene. Particularly preferred disease targets include, without limitation, various cancers. The uses and compositions according to the invention may also be used to activate silenced genes to provide missing gene functions and thus improve a given condition. For example, the uses and compositions of the invention are useful to downregulate and/or suppress abnormal oncogene expression and activity thereby inhibiting tumorigenesis.

In certain embodiments, each of the antisense oligonucleotide and the protein effector is admixed with a pharmaceutically acceptable carrier prior to contact with the cell. In certain embodiments, the antisense oligonucleotide and the protein effector are mixed together prior to contact with the cell.

In certain preferred embodiments of the first aspect of the invention, the cell is to be contacted separately with each of the antisense oligonucleotide and the protein effector. For example, the cell may be contacted with the antisense oligonucleotide prior to being contacted with the protein effector. The cell may be contacted with an effective synergistic amount of one or more antisense oligonucleotides of the invention, followed by contact with an effective synergistic amount of one or more protein effectors of the invention. This is particularly useful where the gene encodes a DNA MeTase and where the to be contacted cell is desired to undergo apoptosis or be arrested in the S phase of the cell cycle.

In another example, the cell may be contacted with the protein effector prior to being contacted with the antisense oligonucleotide. This is particularly useful where the gene encodes a DNA MeTase where the to be contacted cell is desired to be arrested in the G, phase of the cell cycle.

Furthermore, where the gene encodes, for example, DNA MeTase, the uses and compositions according to the invention may also be used to activate silenced genes to provide a missing gene function and thus ameliorate disease symptoms. For example, the diseases beta thalassemia and sickle cell anemia are caused by aberrant expression of the adult beta globin gene or of a mutated gene. Most individuals suffering from these diseases have normal copies of the fetal gene for beta globin. However, the fetal gene is hypermethylated and is silent. Activation of the fetal globin gene could provide the needed globin function, thus ameliorating the disease symptoms. In addition, the uses and compositions according to the invention may be used as gene therapy tools to maintain, activate, and/or modulate the expression of exogenous sequences otherwise liable to inhibition by methylation.

Accordingly, in a certain embodiment of the first aspect of the invention, the gene encodes DNA MeTase. Particularly preferred non-limiting examples of antisense oligonucleotides complementary to regions of RNA or double-stranded DNA encoding DNA MeTase utilized in the use according to the invention are shown in Table 1. DNA MeTase RNA (see *e*.*g*., Yen et al. (1992) Nucl. Acids Res. 9: 2287-2291; Yoder *et al*. (1996) J. Biol. Chem. 271: 31092-31097; Bester *et al*. (1988) J. Mol. Bio. 203(4): 971-983) or double stranded DNA regions include, without limitation, intronic sequences, untranslated 5' and 3' regions, intron-exon boundaries as well as coding sequences from the DNA MeTase gene (see Ramchandani *et al*. (1998) Biol. Chem. 379(4-5): 535-540).

Particularly preferred oligonucleotides have nucleotide sequences of from about 13 to about 35 nucleotides which include the nucleotide sequences shown in Table 1. Yet additional particularly preferred oligonucleotides have nucleotide sequences of from about 13 to about 19 nucleotides of the nucleotide sequences shown in Table 1.

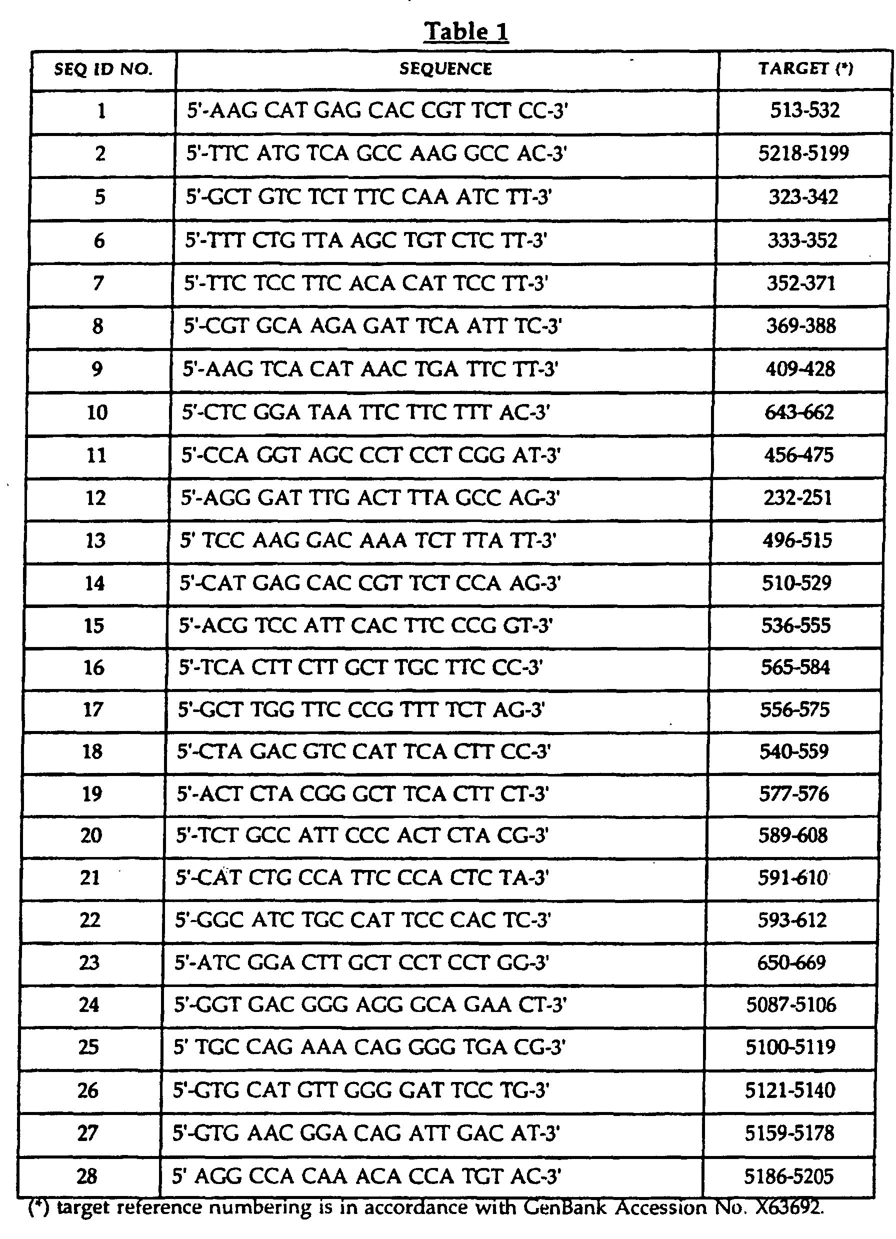

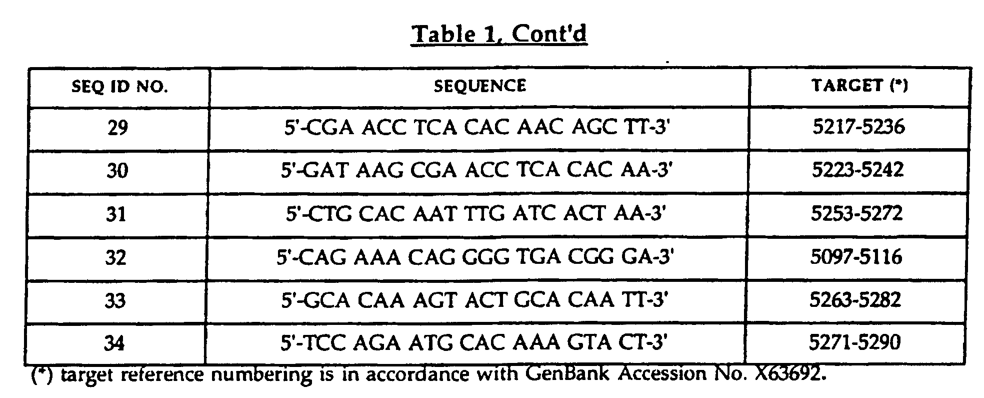

As used herein, a DNA MeTase protein effector preferably inhibits DNA MeTase at least 5-fold, more preferably at least 10-fold, even more preferably at least 50-fold, and most preferably at least 100-fold more than it inhibits any unrelated protein.

In preferred embodiments, the DNA MeTase protein effector is a moiety capable of forming a stable adduct with DNA methyltransferase, thus mimicking the transient covalent intermediate complex believed to be formed during methylation (see, *e*.*g*., Wu and Santi (1987) J. Biol. Chem. 262: 4778-4786). Preferable examples of DNA MeTase protein effectors include without limitation nucleoside analogs such as 5-aza-2'-deoxycytidine (5-aza-dC), 5-fluoro-2'-deoxycytidine, 5-aza-cytidine (5-aza-C), or 5,6-dihydro-5-azacytidine or a pharmaceutically acceptable salt thereof. A method of synthesizing 5,6-dihydro-5-azacytidine from 5'-aza-cytidine is described in U.S. Patent No. 4,058,602 which is hereby incorporated by reference in its entirety. Additional DNA MeTase protein effectors include the inhibitors of DNA methyltransferase enzyme, including hairpin oligonucleotides, described in PCT publication no. WO 97/44346 (PCT application no. PCT/IB97/00879).

In particularly preferred embodiments of all aspects of the invention, the antisense oligonucleotide is in operable association with a protein effector. The term "operable association" includes any association between the antisense oligonucleotide and the protein effector which allows an antisense oligonucleotide to inhibit DNA MeTase gene expression and allows protein effector(s) to inhibit DNA MeTase enzyme activity.

In preferred embodiments of the first aspect of the invention, the invention provides a use of an effective synergistic amount of an oligonucleotide which inhibits DNA MeTase expression and an effective synergistic amount of a DNA MeTase protein effector for the preparation of a pharmaceutical composition for inhibiting DNA MeTase in a cell, wherein the cell is to be contacted with said effective synergistic amounts.

In certain preferred embodiments of the first aspect of the invention, the gene encodes a DNA MeTase and the cell comprises a gene whose expression has been inactivated by methylation. Thus, expression of the gene is promoted and/reactivated in the contacted cell. Preferably, the invention provides compositions and uses for the reactivation of a tumor suppressor gene which has been inactivated by methylation in a cell, such as a neoplastic cell or a cell predisposed to become a neoplastic. By "neoplastic cell," as used herein for all aspects of the invention, is meant a cell that shows aberrant cell growth, such as increased cell growth. A neoplastic cell may be a hyperplastic cell, a cell that shows a lack of contact inhibition of growth *in vitro,* a tumor cell that is incapable of metastasis *in vivo*, or a cancer cell that is capable of metastasis *in vivo*. Any growth of neoplatic cells, whether metastatic or benign, is referred to herein as a tumor or a tumor growth.

In certain preferred embodiments, where the gene encodes DNA MeTase, the invention provides compositions and uses for the reactivation of a tumor suppressor gene which has been inactivated by methylation. In particularly preferred embodiments, the invention provides a use for the reactivation of a p16 tumor suppressor gene which had been inhibited by methylation.

In certain preferred embodiments of this aspect of the invention, where the gene encodes a product not limited to DNA MeTase, uses and compositions are provided for the modulation of target oncogenes such as, for example, the mutant forms of the RAS oncogene family which have been implicated in as many as 75% of human pancreatic cancers (see, *e.g.,* Rodenhuis *et al*. (1992) Semin. Cancer Biol. 3(4): 241-247; Brentnall *et al*. (1995) Cancer Res. 55(19): 4264-4267).

In addition, analogous uses and compositions may also be used to inhibit any number of target genes and/or products of these genes. Consequently, these uses and compositions are useful in therapeutic approaches to various human conditions such as inflammation or asthma as discussed herein.

Accordingly, use the oligonucleotides have nucleotide sequences of from about 15 to about 26 nucleotides of the nucleotide sequences shown for illustrative purposes in Tables 2 and 3.

Table 2
SEQ ID NO. | SEQUENCE | TARGET (**)
35 | 5'-GAG ACA GCA GCA CCA GCG GG -3' | 17-36
36 | 5'-ATG ACC GAG TCG GAG ACA GC-3' | 21-49
37 | 5'-GGA TGA CCG AGT GGG AGA CA-3' | 31-50
38 | 5'-CAG GAT GAC CGA GTG GGA GA -3' | 33-52
39 | 5'-TGT GTT CTC AGG ATG ACC GA-3' | 41-60
40 | 5'-GAG TGA CAG AGA CGC TCA GG-3' | 62-81
41 | 5'-TTC TGG CTT CTC CTC CTT GG-3' | 1504-1523
42 | 5'-CTT GAC CTC CTC CTT GAC CC-3' | 1531-1550
43 | 5'-GGA AGC CAG AGC TGG AGA GG-3' | 1565-1584
44 | 5'-GAA ACG TGA GGG ACT CAG CA-3' | 1585-1604
45 | 5'-CCG TCG TAG TAG TAA CAG ACT TT-3' | 138-160
46 | 5'-TGT CCA TAA TAG TAA TTT CCA A-3' | 166-187
47 | 5'-CAG CAA ATT ATG AGT CAT GCG GAT TC-3' | 211-236
(**) target reference numbering is in accordance with HDAC-1, GenBank Accession Number
U50079.

Table 3
SEQ ID NO. | SEQUENCE | TARGET (***)
50 | 5'-CTC CTT GAC TGT ACG CCA TG-3' | 1-20
51 | 5'-TGC TGC TGC TGC TGC TGC CG-3' | 121-141
52 | 5'-CCT CCT GCT GCT GCT GCT GC-3' | 132-152
53 | 5'-CCG TCG TAG TAG TAG CAG ACT TT-3' | 138-160
54 | 5'-TGT CCA TAA TAA TAA TTT CCA A-3' | 166-187
55 | 5'-CAG CAA GTT ATG GGT CAT GCG GAT TC-3' | 211-236
56 | 5'-GGT TCC TTT GGT ATC TGT TT-3' | 1605-1625
(***) target reference numbering is in accordance with HDAC-2, GenBank Accession
Number U31814.

In a second aspect, the invention provides a use of an inhibitor for the preparation of a pharmaceutical composition for treating a disease responsive to inhibition of a gene. In the use according to this aspect of the invention an antisense oligonucleotide which inhibits expression of the gene, and a therapeutically effective synergistic amount of a protein effector of a product of the gene is to be administered to a mammal, including a human, which has at least one cell affected by the disease. The antisense oligonucleotide and the protein effectors are as described for the first aspect according to the invention. "A disease responsive to inhibition of a gene" is one which is associated with altered activity, levels, or functions of the gene and/or a product of the gene. The symptoms of such a disease are alleviated and/or eliminated by the modulation of the activity of the gene or of a product of the gene. "A cell affected by the disease" is a cell which has altered activity, levels, or functions of the gene and/or a product of the gene.

For example, "a disease responsive to DNA MeTase inhibition" is one which is associated with altered methylation pattern(s) or altered DNA MeTase activity, levels, or functions. The symptoms of such a disease are alleviated and/or eliminated by the modulation of DNA MeTase activity. "A cell affected by a disease responsive to DNA MeTase inhibition" is a cell which has altered methylation pattern(s) or altered DNA MeTase activity, levels, or functions.

In certain embodiments of the second aspect of the invention, the mammal is to be administered a therapeutically effective synergistic amount of at least one antisense oligonucleotide and/or at least one protein effector for a therapeutically effective period of time.

The terms "therapeutically effective synergistic amount" and "therapeutically effective period of time" are used to denote treatments at dosages and for periods of time effective to achieve the therapeutic result sought. The therapeutically effective synergistic amount of the antisense oligonucleotide and/or the therapeutically effective synergistic amount of the protein effector is/are less than the amount(s) empirically found necessary to inhibit the gene when either the antisense oligonucleotide or the protein effector are used individually. In preferred embodiments, the combined inhibitory effect of the antisense oligonucleotide and the protein effector according to the invention are more than additive, *i*.*e*.,the combined inhibitory effect is greater than the expected total effect calculated on the basis of the sum of the individual effects. One of skill in the art will appreciate that such synergistic effect resulting in a lower effective concentration of either the antisense oligonucleotide, the protein effector or both may vary considerably depending on the tissue, organ, or the particular mammal or patient to be treated according to the invention. Furthermore, one of skill will appreciate that the therapeutically effective synergistic amount of either the antisense oligonucleotide or the protein inhibitor may be lowered or increased by fine tuning and altering the amount of the other component. The invention therefore provides a use to tailor the administration/treatment to the particular exigencies specific to a given mammal or patient. As illustrated in the following examples, therapeutically effective synergistic ranges may be easily determined, for example, empirically by starting at relatively low amounts and by step-wise increments with concurrent evaluation of inhibition. In particularly preferred embodiments, the therapeutic composition of the invention is to be administered systemically at a sufficient dosage to attain a blood level of antisense oligonucleotide from about 0.01µM to about 20µM and of protein effector from about 0.01µM to about 10µM. In particularly preferred embodiments, the therapeutic composition is to be administered at a sufficient dosage to attain a blood level of antisense oligonucleotide from about 0.05µM to about 15µM and of protein effector from about 0.05µM to about 10µM. In even more preferred embodiments, the blood level of antisense oligonucleotide is from about 0.1µM to about 10µM and the blood level of protein effector is from about 0.1µM to about 7µM. For localized administration, much lower concentrations than this may be effective. Preferably, a total dosage of antisense oligonucleotide will range from about 0.1 mg to about 30 mg oligonucleotide per kg body weight per day, while, a total dosage of protein effector will range from about 0.01 mg to about 5 mg protein effector per kg body weight per day. In more preferred embodiments, a total dosage of antisense oligonucleotide will range from about 1 mg to about 20 mg oligonucleotide per kg body weight per day, while, a total dosage of protein effector will range from about 0.1 mg to about 4 mg protein effector per kg body weight per day. In most preferred embodiments, a total dosage of antisense oligonucleotide will range from about 2 mg to about 10 mg oligonucleotide per kg body weight per day, while, a total dosage of protein effector will range from about 0.1 mg to about 1 mg protein effector per kg body weight per day. In particularly preferred embodiments, the therapeutically effective synergistic amount of antisense oligonucleotide is 0.5 mg oligonucleotide per kg body weight per day, and the effective synergistic amount of protein effector is 0.1 mg per kg body weight per day.

In certain embodiments, each of the antisense oligonucleotide and the protein effector is admixed with a pharmaceutically acceptable carrier prior to administration to the mammal. In certain embodiments, the antisense oligonucleotide and the protein effector are mixed together prior to administration to the mammal.

Each of the antisense oligonucleotides and the protein effectors according to all aspects of the invention may optionally be formulated with any of the well known pharmaceutically acceptable carriers or diluents. Pharmaceutically acceptable carriers and their formulations are well-known and generally described in, for example, Remington's Pharmaceutical Sciences (18th Edition, ed. A. Gennaro, Mack Publishing Co., Easton, PA, 1990). Formulations of the invention may further contain one or more DNA MeTase inhibitors and/or one or more additional DNA MeTase antisense oligonucleotide(s), and/or one or more protein effector(s), or it may contain any other pharmacologically active agent. If an antisense oligonucleotide is to be administered with a protein effector, both may be admixed together with a pharmaceutically acceptable carrier. Where the antisense oligonucleotide is administered separately from the protein effector, each may be mixed with a pharmaceutically acceptable carrier. It will understood that where the antisense oligonucleotide and the protein effector are to be administered separately, the same pharmaceutically acceptable carrier need not be the same for both. Rather, the pharmaceutically acceptable carrier is dependent on the route of administration of the antisense oligonucleotide and of the protein effector.

The compositions of the invention may be administered by any appropriate means. For example, the compositions of the invention may be administered to an mammal within a pharmaceutically-acceptable diluent, carrier, or excipient, in unit dosage form according to conventional pharmaceutical practice. Administration may begin before the mammal is symptomatic for a disease responsive to inhibition of a gene. For example, where the disease is responsive to DNA MeTase inhibition, such as cancer, administration may begin before the animal is symptomatic.

Any appropriate route of administration may be employed, including, without limitation, parenteral intravenous, intra-arterial, subcutaneous, sublingual, transdermal, topical, intrapulmonary, intramuscular, intraperitoneal, by inhalation, intranasal, aerosol, intrarectal, intravaginal, or by oral administration. Therapeutics may be in the form of liquid solutions or suspensions; for oral administration, formulations may be in the form of tablets or capsules; and for intranasal formulations, in the form of powders, nasal drops, or aerosols. The compositions may be administered locally to the area affected by a disease responsive to inhibition of a gene. For example, where the disease is responsive to DNA MeTase inhibition and is a cancer, the composition may be administered directly into the tumor mass): The compositions of the invention may be administered systemically.

In certain preferred embodiments of the second aspect of the invention, the antisense oligonucleotide and the protein effector are to be administered separately to the mammal. For example, the antisense oligonucleotide may be administered to the mammal prior to administration to the mammal of the protein effector. The mammal may receive one or more dosages of antisense oligonucleotide prior to receiving one ore more dosages of protein effector. Where the gene encodes a DNA MeTase, this administration schedules particutarly useful where the cell affected by the disease is desired to undergo apoptosis or be arrested in the S phase of the cell cycle. Such an administration schedule may be useful, for example, where the gene encodes DNA MeTase and where the disease is an aggressive cell proliferative disease such as metastatic cancer.

In another example, the protein effector may be administered to the mammal prior to administration of the antisense oligonucleotide. The mammal may receive one or more dosages of protein effector prior to receiving one or more dosages of antisense oligonucleotide. Where the gene encodes a DNA MeTase, this administration schedule is particularly useful where the cell affected by the disease is desired to be arrested in the G, phase of the cell cycle. Such an administration schedule may be useful, for example, where the gene encodes DNA MeTase and where the disease is associated with cells whose growth arrest, rather than death, is desired. One non-limiting example of such a disease is transplantation graft rejection, where the host's immune cells (e.g., lymphocytes and leukocytes) proliferate in response to the foreign cells in the transplanted graft. Arrest of growth of the host's immune cells, rather than the death of these cells by apoptosis, is desirable.

In certain preferred embodiments of the second aspect of the invention, the gene encodes a DNA MeTase and the cell affected by the disease comprises a gene whose expression has been inactivated by methylation. In certain embodiments, expression of the gene is promoted, restored, and/or reactivated in the cell in the mammal to which has been administered a therapeutically effective synergistic amount of an antisense oligonucleotide and a therapeutically effective synergistic amount of a protein effector. In preferred embodiments, the gene is the p16^{ink4} tumor suppressor gene.

In certain embodiments of this aspect of the invention, the gene encodes a DNA methyltransferase. In certain embodiments, the protein effector is selected from the group consisting of 5-aza-cytidine, 5-aza-2'-deoxycytidine, 5-fluoro-2'-deoxycytidine and 5,6-dihydro-5-azacytidine.

In a third aspect, the invention provides a use of an inhibitor for the preparation of a pharmaceutical composition for inhibiting tumor growth in a mammal. In the use according to this aspect of the invention a therapeutically effective synergistic amount of an antisense oligonucleotide which inhibits expression of a gene involved in tumorigenesis, and a therapeutically effective synergistic amount of a protein effector of a product of the gene is to be administered to a mammal, including a human, which has at least one neoplastic cell present in its body. As used herein, "a gene involved in tumorigenesis," is a gene whose aberrant expression is associated with tumorigenesis. Exemplary genes involved in tumorigenesis include the genes encoding DNA methyltransferases. By "tumorigenesis" is meant the genetic and phenotypic events involved in the progression of a normal cell to become a neoplastic cell. The antisense oligonucleotide and the protein effectors are as described for the first aspect according to the invention. Administration and dosages are as described for the second aspect according to the invention.

The gene encodes a DNA methyltransferase. In certain embodiments, the protein effector is selected from the group consisting of 5-aza-cytidine, 5-aza-2'-deoxycytidine, 5-fluoro-2'-deoxycytidine and 5,6-dihydro-5-azacytidine.

In embodiments of the third aspect of the invention, the mammal is to be administered a therapeutically effective synergistic amount of at least one antisense oligonucleotide and/or at least one protein effector for a therapeutically effective period of time. The terms, "therapeutically effective synergistic amount" and "therapeutically effective period of time," are as described in the second aspect of the invention. In certain embodiments of the third aspect, each of the antisense oligonucleotide and the protein effector is admixed with a pharmaceutically acceptable carrier prior to administration to the mammal. In certain embodiments, the antisense oligonucleotide and the protein effector are mixed prior to administration to the mammal.

In certain preferred embodiments of the third aspect of the invention, the the antisense oligonucleotide and the protein effector are to be separately administered to the mammal. For example, the antisense oligonucleotide may be administered to the mammal prior to the administration of the protein effector. This is desirable where the gene encodes a DNA MeTase and where the neoplastic cell in the mammal is desired to undergo apoptosis or be arrested in the S phase of the cell cycle. This administration schedule is particularly useful, for example, in the treatment of an aggressive, metastatic tumor such as melanoma.

Alternatively, the protein effector may be administered to the mammal prior to the administration of the antisense oligonucleotide. This schedule is desirable where the gene encodes a DNA MeTase and where the neoplastic cells in the mammal is desired to be growth arrested in the G, phase of the cell cycle. This administration schedule is particularly useful, for example, in the treatment of slower growing tumors (*e*.*g*., prostate cancer) or in the treatment of infirmed patients where the presence of apoptotic cells is not desirable.

In certain preferred embodiments of the third aspect of the invention, where the gene encodes a DNA MeTase, the neoplastic cell further comprises a gene whose expression has been inactivated by methylation. In certain embodiments, expression of the gene is promoted, restored, and/or reactivated in the neoplastic cell in the mammal to which has been administered a therapeutically effective synergistic amount of an antisense oligonucleotide and a therapeutically effective synergistic amount of a protein effector. In preferred embodiments, the gene is the p16^{ink4} tumor suppressor gene.

In a fourth aspect, the invention provides an inhibitor of a gene comprising an antisense oligonucleotide which inhibits expression the gene in operable association with a protein effector of a product of the gene. In certain embodiments of this aspect of the invention, the antisense oligonucleotide is in operable association with two or more protein effectors.

The antisense oligonucleotide and protein effector of this aspect of the invention as as described for the first aspect according to the invention.

In certain embodiments of the fourth aspect of the invention, the gene encodes a DNA methyltransferase. In certain embodiments, the protein effector is selected from the group consisting of 5-aza-cytidine, 5-aza-2'-deoxycytidine, 5-fluoro-2'-deoxycytidine and 5,6-dihydro-5-azacytidine.

In a fifth aspect, the invention provides a pharmaceutical composition comprising an inhibitor of a gene comprising an antisense oligonucleotide in operable association with a protein effector. In certain embodiments, the composition further comprises a pharmaceutically acceptable carrier.

In a sixth aspect, the invention provides a use of an inhibitor for the preparation of a pharmaceutical composition for investigating the role of a gene and/or a product of that gene in cellular growth, including the growth of tumor cells. In the use according to this aspect, the cell type of interest (*e*.*g*., a neoplastic cell) is to be contacted with a synergistic amount of an antisense oligonucleotide which inhibits expression of the gene and a synergistic amount of a protein effector of a product of the gene, as described for the first aspect according to the invention, resulting in inhibition of expression of the gene in the cell. The combinations described herein may be administered at different points in the cell cycle (*e*.*g*., at the G₁ phase, S phase, or G₂/M phase), or in conjunction with promoters or inhibitors of cell growth to determine the role of the gene (*e*.*g*., the gene encoding DNA MeTase) in the growth of the cell type of interest. In certain embodiments, the cell is a neoplastic cell.

The following examples are intended to further illustrate certain preferred embodiments of the invention and are not limiting in nature. Those skilled in the art will recognize or be able to ascertain, using no more than routine experimentation, numerous equivalents to the specific substances and procedures described herein. Such equivalents are considered to be within the scope of this invention, and are covered by the following claims. Examples 8-10 are shown only for illustrative purposes.

### Example 1

### Selection of Antisense Oligonucleotides Capable of Inhibiting MeTase Expression in Neoplastic cells

To identify antisense oligodeoxynucleotides capable of inhibiting DNA MeTase gene expression in human neoplastic cells, 85 phosphorothioate oligodeoxynucleotides (each 20 base pairs in length) bearing sequences complimentary to the 5' and 3' regions of human DNA MeTase mRNA and sequences complimentary to intron-exon boundaries were synthesized and screened for antisense activity. As shown in Fig. 1, the two DNA MeTase mRNA regions were identified to be highly sensitive to antisense inhibition were targeted by MG88 having the sequence 5'-AAG CAT GAG CAC CGT TCU CC-3' (SEQ ID NO:48) (this oligonucleotide is targeted to the DNA MeTase mRNA 5'UTR at nucleotides 532 to 513) and MG98 having the sequence 5'-UUC ATG TCA GCC AAG GCC AC-3' (SEQ ID NO:49) (this oligonucleotide is targeted to the DNA MeTase mRNA 3'UTR at nucleotides 5218 to 5199). These oligonucleotides were chemically modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of each oligonucleotide consisted of a phosphorothioate linkage between adjoining nucleotides. MG88 and MG98 have IC₅₀ values of 40 nM and 45 nM for inhibition of DNA MeTase mRNA, respectively.

MG88 was next tested for an ability to inhibit DNA MeTase mRNA in two human neoplastic cells, A549 (human non-small cell lung carcinoma cells) and T24 (human bladder cancer cells). Cells were transfected with lipofectin only (6.25 µg/ml), or lipofectin plus 20, 40, or 80 nM of MG88 or control oligonucleotide MG208 having the sequence 5'-AAC GAT CAG GAC CCT TGU CC-3' (SEQ ID NO:4). MG208 was modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of MG208 consisted of a phosphorothioate linkage between adjoining nucleotides.

The MG88 and MG208 oligonucleotides were diluted to the desired concentration from a 0.1 mM stock solution in the transfection media. Cells were exposed to oligonucleotide plus lipofectin (or lipofectin only) for four hours on day 0 and four hours on day 1 (after each four hour treatment, cells were returned to complete media). On day 2 *(i.e.,* 48 hours after the first treatment on day 0), cells were harvested, whole cell lysates were prepared, and DNA MeTase protein levels were analyzed by Western blotting analysis with a DNA MeTase-specific rabbit polyclonal antibody (generated according to standard techniques of a glutathione S-transferase fusion protein containing the first 10 kDa amino terminus from DNA methyltransferase protein). As shown in Fig. 2, treatment with MG88, but not control MG208, produced dose-dependent reduction in DNA MeTase protein levels. Equal loading of all lanes was confirmed by blotting for α-actin protein levels with an actin-specific rabbit polyclonal antibody (commercially available from Santa Cruz Biotech., Santa Cruz, CA).

### Example 2

### Inhibition of the p16 Tumor Suppressor by targeting DNA MeTase

The cyclin-dependent kinase inhibitor (CDKI) p16^{ink4A} regulates the transition from G, to S-phases of the cell cycle (Serrano *et al*. (1993) Nature 366: 704-707). Inactivation of p16^{ink4A} is one of the most frequently observed abnormalities in human cancer (Serrano *et al*., *supra).* Transcriptional inactivation and associated hypermethylation of the p16^{ink4A} promote region have also been observed in virtually all types of cancer (Gonzales-Zulueta *et al*. (1995) Cancer Res. 55: 4531-4535; Merlo *et al*. (1995) Nat. Med. 7: 686-692; Costello *et al*. (1996) Cancer Res. 56: 2405-2410; Lo *et al*., Cancer Res. 56: 2721-2725). To investigate the effect of specifically reducing cellular DNA MeTase levels on the expression and methylation status of a silenced p16^{ink4A} gene, T24 human bladder cancer cells that contain a hypermethylated and silenced p16^{ink4A} gene were transfected with 40 nM or 75nM MG88 or control oligonucleotide MG208 with 6.25 µg/ml lipofectin for four hours each day for up to 10 consecutive days. On days 3, 5, 8, and 10, p16^{ink4A} protein levels were then analyzed by immunoprecipitation followed by Western blotting analysis with a p16^{ink4A} protein-specific antibody (PharMingen). HeLa cells were used as a positive (+) control for p16^{ink4A} protein expression.

As shown in Fig. 3A, induction of expression of p16^{ink4A} protein was detected after 5 days of treatment with either 40 nM or 75 nM of MG88, but not after treatment with MG208. Because MG88 has an antiproliferative effect, p16^{ink4A} levels were normalized to cell number (Fig. 3B), Thus, induction of p16^{ink4A} protein by MG88 was both dose-dependent and time dependent. p16^{ink4A} was not detected in cells treated with either 40 nM or 75 nM of the mismatch control MG208 or lipofectin alone (Fig. 3A).

Moreover, reactivation of p16^{ink4A} protein expression by MG88 also caused accumulation of hypophosphorylated pRb and inhibition of cell proliferation. p16^{ink4A} regulation progression through the G₁ phase of the cell cycle by inhibiting cyclin-dependent kinase CDK4-mediated phosphorylation of pRB such that the hypophosphrylated form of Rb is associated with G₁/G₀ growth arrest (Serrano *et al*., *supra*). Cell lysates of T24 cells which were reactivated to express p16^{ink4A} protein following transfection with MG88 were analyzed by Western blotting analysis for phosphorylated pRb showed a decrease in the amount of phosphorylated forms of pRb, thus increasing the relative abundance of the hypophosphorylated form of pRb (Fig. 4). These results demonstrate that high levels of DNA MeTase in T24 cells actively suppress p16^{ink4A} gene expression and that restoration of p16^{ink4A} expression functionality regulations downstream molecular targets, such as pRb.

Next, to determine whether *de novo* methylation and silencing of the re-expressed p16^{ink4A} gene occurred when DNA MeTase returned to normal levels, T24 cells were transfected for 10 consecutive days with either 40 or 75 nM or MG88 or MG208. Because transfection of the cells with 75 nM MG88 had a significant antiproliferative and cell death inducing effect, only cells treated with the lower dose (40 nM) of MG88 were used for the remainder of the experiment. Cell lysates of the T24 cells transfected for ten days were analyzed by Western blotting analysis for expression of DNA MeTase protein,p16^{ink4A} protein, and actin protein (as a control for equal loading) 3, 5, and 7 days after MG88 transfection. As shown in Fig. 5, DNA MeTase protein levels increased in the absence of MG88 treatment and returned to control levels between days 5-7 post-treatment (middle panel). The level of p16^{ink4A} protein decreased steadily over the post-treatment period until it was barely detectable at day 7 post-treatment (Fig. 5, upper panel). Fig. 6 shows the inverse relationship between DNA MeTase levels and p16^{ink4A} protein levels during and after treatment with MG88. Interestingly, the loss of p16^{ink4A} protein expression began at day 14 after DNA MeTase levels returned to near control levels. This lag suggests that elevated levels of DNA MeTase over several rounds of replication are required to methylate and inactivate p16^{ink4A} gene expression. That the inactivation and *de novo* methylation of p16^{ink4A} are coincident with elevated levels of the DNA MeTase (Dnmt1) suggests that it may contribute to *de novo* methylation activity itself.

To identify changes in the methylation status of the p16^{ink4A} promoter induced by MG88 treatment, methylation specific PCR (MSP) and bisulfite genomic sequencing was performed as previously described (Caldas *et al*. (1994) Nat. Gen. 8: 27-32; Frommer *et al*. (1991) Proc. Nat. Acad. Sci. USA 89: 1827-2831). MSP of the p16^{ink4A} promoter was performed on T24 cells treated with 40µM or 75µM of MG88 or MG208 for 3, 5, 9, or 10 days. Briefly, the Oncor p16 detection system was used (commercially available from Oncor, Gaithersburg, MD). PCR was performed in a total volume of 25 µl under the following conditions: 100 ng bisulfate-treated DNA (Oncor),10 mM Tris-HCl, pH 8.3, 50 nM KCl, 1.5 mM MgCl₂, 250 µM dNTPs, 80 ng of each of the following primers (5'-GTAGGTGGGGAGGAGTTTAGTTT-3' sense (SEQ ID NO: 79) and 5'-TCTAATAACCAACCAACCCCTAA-3' antisense (SEQ ID NO: 80)) and 1 unit of AmpliTaq Gold (Perkin-Elmer). The denaturation cycle was 95°C for 12 min followed by 35 cycles at 95°C for 45 seconds, 60°C for 45 seconds, and 72°C for 60 seconds, and an elongation cycle of 72°C for 10 minutes. the PCR product (5µl) was analyzed on a 2% agarose gel. The unmethylated (U) and demethylated (D) primers (commercially available from Oncor) were used at the same conditions as the specific primers. PCR products were subcloned into PCR2.1 (commercially available form Invitrogen, Carlsbad, CA), and sequenced to determine demethylation of CpG sites in the p16 proximal promoter.

As shown in Fig. 7, MSP analysis with PCR primers specific for methylated p16^{ink4A} (M) or unmethylated p16^{ink4A} (U) revealed that demethylation of the p16^{ink4A} promoter region occurred as early as day 3 of MG88 treatment. Treatment with MG208 or lipofectin alone had no effect on methylation of the p16^{ink4A} gene (Fig. 7). Fig. 8 shows that by employing bisulfite genomic sequencing on days 0, 3, and 5 of treatment with MG88 or MG208, fifteen CpG sites within the p16^{ink4A} promote were found to be methylated in untreated T24 cells. Inhibition of the DNA MeTase by MG88 led to demethylation of 5 of fifteen CpG sites by day 3 and demethylation at all 15 CpG sites by day 5 of treatment, whereas treatment with the control MG208 had no effect on p16^{ink4A} methylation status (Fig. 8). Three days after cessation of MG88 treatment the p16^{ink4A} promoter shows significant re-methylation at 13 of 15 sites reflecting either *de novo* methylation of these sites or a rapid expansion of a less affected population (Fig. 8, bottom panel).

To study the effect of specific inhibition of DNA MeTase on cell growth, cellular proliferation rates were monitored both during the treatment and post treatment periods to determine the duration of the effect. T24 cells were treated for 0-5 days with lipofectin only, 75 nM MG88 or 75 nM MG208. During and following treatment, cells were counted. As can be seen in Fig. 9B, during the course of treatment, MG88 dramatically inhibited cell proliferation, whereas treatment of cells with the control MG208 caused only minimal growth inhibition relative to lipofectin treated cells. Inhibition of cell proliferation persisted for approximately one week post-treatment, consistent with the finding that p16^{ink4A} expression was maintained until 7 days after the last dose of MG88 (see Figs. 5 and 6). To determine whether the transient re-expression of p16^{ink4A} and growth-inhibitory effects induced by short term treatment with MG88 were due to the expansion of a less affected (less demethylated) population of cells within the treated population, or to rapid inactivation of p16^{ink4A} after MG88 withdrawal, single cell clones were isolated after treatment. Several MG88 clones were, in fact, p16^{ink4A} negative, confirming that the MG88 treatment produced a mixed population of p16^{ink4A} positive and negative cells. Methylation analysis by bisulfite sequencing of the p16^{ink4A} promoter of one of the MG88 positive clones, clone 4-5, thirty days following treatment with a five day treatment with MG88 revealed that the p16^{ink4A} promoter region was completely non methylated at all CpG sites evaluated (Fig. 9A), demonstrating that even short term (5 day) inhibition of the DNA MeTase by MG88 could induce prolonged re-expression of a silenced tumor suppressor gene. As shown in Figs. 9B and 9C, clone MG88 C4-5 initially grew slowly following a five day treatment with MG88 as compared to lipofectin clone C-5 (treated for 5 days with lipofectin) and MG208 C2-4 (treated for 5 days with MG208); however between days 40 and 45 days post treatment, the growth rate of MG88 C4-5 cells increased dramatically (Fig. 9C). Determination of p16^{ink4A} protein levels in MG88 C4-5 cells revealed a significant decrease at Day 49 (Fig. 9C, inserted Western blotting analysis). Loss of p16^{ink4A} expression after prolonged culture in the absence of MG88 treatment suggests that the DNA MeTase targeted (thought to encode maintenance DNA MeTase activity) may have *de novo* methyltransferase activity and over time can methylate and inactivate previously unmethylated actively expressing genes.

### Example 3

### Inhibition of MeTase Rapidly Induces p21^{WAF1}

Another member of the cyclin-dependent kinase inhibitor (CDKI) family, p21^{WAF1}, inhibits a wide range of cyclin/CDK complexes involved in G₁ and S phase progression (Tam *et al*. (1994) Cancer Res. 54: 5816-5820; Baghdassarian and French (1996) Hematol. Cell Ther. 88: 313-323; Gotz *et al*. (1996) Oncogene 13: 391-398). To investigate whether DNA MeTase and p21^{WAF1} protein levels are linked by a regulatory pathway, p21^{WAF1} protein levels were measured in T24 cells in which DNA MeTase levels had been incrementally reduced by MG88 treatment. To do this, DNA MeTase, p21^{WAF1}, and α-actin protein levels were measured by Western blotting analysis in T24 cells that had been treated for either 24 hours (*i*.*e*., one 4 hour transfection time) or 48 hours (*i.e,* two 5 hour transfections 24 hours apart) with either 40nM or 75nM of either MG88 or MG208. As shown in Fig. 10A, p21^{WAF1} increased directly with the reduction in DNA MeTase, while neither lipofectin nor MG208 had an effect on either DNA MeTase or p21^{WAF1} levels.

T24 cells were also treated for 24 hours (*i*.*e*., four hour transfection) with lipofectin only, or lipofectin plus 20, 40, or 80 nM of MG88 or MG208. After treatment, cell lysates were prepared and analyzed by Western blotting analysis with α-actin-specific antibody or p21-specific antibody.. As shown in Fig. 10B, DNA MeTase inhibition induced p21^{WAF1} in a dose dependent fashion as early as 24 hours after MG88 treatment, consistent with a role for p21^{WAF1} in the antiproliferation effect observed with MG88 treatment (see, *e.g.,* Figs. 3B and 9B).

Next, to determine if p21^{WAF1} was induced at the transcriptional level, RNase protection assay were performed on cells treated with 40 nM of either MG88 or MG208 for either 24 or 48 hours. Total cellular RNA was isolated from the cells and analysed for p21 mRNA, p27 mRNA, and p18 mRNA using the human cell cycle-2 (hcc-2) multiprobe RNase protection kit commercially available from PharMingen, San Diego, CA). RNA loading was determined to be equal using the signals from two housekeeping genes, L32 and GAPDH. As shown in Fig. 11, no increase in p21^{WAF1} mRNA was observed in response to treatment with MG88, suggesting that post-translational regulation of p21^{WAF1} protein is involved.

This example demonstrates that a functional antagonism between DNA MeTase and p21^{WAF1} on cellular proliferation exists. Thus, high levels of DNA MeTase found in transformed cells may regulate proliferation by reducing cellular p21^{WAF1} levels. High levels of DNA MeTase in transformed cell may also compete directly for the downstream target PCNA, since human DNA MeTase can compete with p21^{WAF1} for PCNA binding (Chuang *et al*. (1997) Science 277: 1996-2000).

### Example 4

### Synergistic Reactivation of the p16 Tumor Suppressor

The purpose of this example is to illustrate the ability of the methods and compositions of the invention to restore the expression of genes which are inactivated by methylation such as, for example, the p16 tumor suppressor as illustrated herein. For this purpose, one day before transfection, T24 cells (ATCC No. HTB-4) were plated onto 10 cm plates at 4 X 10⁵ cells/dish. At the time of transfection, cells were washed with phosphate buffered saline (PBS) and 5 ml of eptimem media (Gibco-BRL) containing 6.25 µl/ ml lipofectin transfection reagent (Gibco-BRL) was added. The oligonucleotides used were: MG88 having the sequence 5'-AAG CAT GAG CAC CGT TCU CC-3' (SEQ ID NO:48) (this oligonucleotide is targeted to the DNA MeTase mRNA 5'UTR at nucleotides 532 to 513) and MG98 having the sequence 5'-UUC ATG TCA GCC AAG GCC AC-3' (SEQ ID NO:49) (this oligonucleotide is targeted to the DNA MeTase mRNA 3'UTR at nucleotides 5218 to 5199). Negative controls used were: MG207 having the sequence 5'-UUA ATG TAA CCT AAG GUC AA-3' (SEQ ID NO:3) and MG208 having the sequence 5'-AAC GAT CAG GAC CCT TGU CC-3' (SEQ ID NO:4). These oligonucleotides were chemically modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of each oligonucleotide consisted of a phosphorothioate linkage between adjoining nucleotides. The oligonucleotides were diluted to the desired concentration from a 0.1 mM stock solution in the transfection media. After a four-hour incubation at 37°C in a 5% CO₂ incubator, the plates were washed with PBS and 10 ml of fresh cell culture media was added. Following an additional two-hour incubation at 37°C, freshly prepared 5-aza-2'-deoxycytidine was added to the tissue culture medium. Cells were transfected for a total of three days and split every other day to ensure optimal transfection conditions. At the indicated time points, cells were harvested by trypsinization and pelleted by centrifugation at 1100 rpm and 4°C for five minutes. The cell pellet was resuspended in PBS and counted on a Coulter Particle Counter to determine the total cell number. Following a second centrifugation, the PBS was aspirated from the cell pellet and the pellet was frozen at 70°C.

Cell pellets were resuspended in 200 µl of cell lysis buffer (25 mM Tris pH 7.5, 5 mM EDTA, 0.5% sodium deoxycholate, and 1% triton X 100) supplemented with protease inhibitors: aprotinin, leupeptin, pepstatin, TLCK, and PMSF. The lysed cells were incubated on ice for 10 minutes and cell particulate matter was removed by centrifugation at 10,000 rpm for 10 minutes at 4°C. The protein concentration for each sample was determined using the Bio Rad Protein Assay and 600 µg of protein was used for each immunoprecipitation. Anti-p16 antibody (Santa Cruz) was added, 2.5 µg/ml, and each sample was incubated at 4°C for one hour on a rotary shaker. Each sample was incubated for an additional 45 minutes at 4°C after the addition of 20 µl of equilibrated Protein G and washed three times with lysis buffer containing no protease inhibitors. The Protein G pellet was resuspended in 15 µl of 2X gel loading buffer, containing β-mercaptoethanol and incubated at room temperature for 10 minutes. After boiling for five minutes, samples were separated by gel electrophoresis on a 4-20% polyacrylamide gradient gel and blotted onto PVDF membrane (Amersham Life Sciences, Cleveland, Ohio). Each membrane was incubated overnight 1X TBST wash buffer (10 mM Tris pH 8.0, 150 mM NaCl, 0.05% Tween 20) supplemented with 5% milk. The membranes were incubated with mouse anti-human p16 antibody (Pharmingen Mississauga, Ontario), 1 µg/ml, for sixty minutes at room temperature. Following three washes in 1X TBST, and a one-hour incubation with the secondary antibody, goat anti-mouse IgG, membranes were washed two times in 1X TBST and two times in 1X TBS, and chemiluminescence was performed. Fig. 12 shows the Western blot analysis of T24 cells treated with various concentrations of 5-aza-dC from 0.1 µM to 0.75 µM, showing reactivation of p16 in the range of 0.3 µM to 0.75 µM 5-aza-dC. Fig. 13 shows the Western blot analysis of T24 cells treated by the method of the invention using antisense oligonucleotide MG88 and 5-aza-dC. A comparison of the bands presence and intensity in Fig. 13 shows that reactivation of the p16 gene is successfully achieved using a combination of the oligonucleotide and the protein effector according to the invention at concentrations at which neither the oligonucleotide nor the protein effector used alone would be effective (e.g., at as low as 40 nM oligonucleotide MG88 and 0.1 µM 5-aza-dC).

Fig. 14 shows the Western blot analysis of T24 cells treated by the method of the invention using antisense oligonucleotide MG98 and 5-aza-dC. Once again, a comparison of the bands presence and intensity in Fig. 14 shows that reactivation of the p16 gene is successfully achieved using a combination of the oligonucleotide and the protein effector according to the invention at concentrations at which neither the oligonucleotide nor the protein effector used alone would be effective (*e.g.*, at as low as 40 nM oligonucleotide MG98 and 0.1 µM 5-aza-dC).

The Western blotting analysis in Fig. 14 was subjected to densitometric analysis and normalized to the level of p16 expression in HeLa cells. The results of the densitometric analysis is shown in Table 5.

**Table 5**

| Treatment of T24 cells | Percentage of HeLa Cell Control |
|---|---|
| (HeLa cell control) | 100 |
| Lipofectin only | 6 |
| Lipofectin plus 0.1 µM 5-aza-dC | 11 |
| 40 nM MG207 | 1 |
| 40 nM MG207 plus 0.1 µM 5-aza-dC | 3 |
| 40 nM MG98 | 4 |
| 40 nM MG98 plus 0.1 µM 5-aza-dC | 79 |

As Table 5 shows, the combination of both MG98 plus 5-aza-dC has a much greater effect on p16 reactivation in T24 cells as compared to either MG98 or 5-aza-dC alone.

Fig. 15 shows the Western blot analysis T24 cells treated by the method of the invention using antisense oligonucleotide MG88 and 5-aza-dC using even lower concentrations of oligonucleotide MG88. A comparison of the bands presence and intensity in Fig. 15 shows that reactivation of the p16 gene is successfully achieved using a combination of the oligonucleotide and the protein effector according to the invention at concentrations at which neither the oligonucleotide nor the protein effector used alone would be effective (*e*.*g*., at as low as 20 nM oligonucleotide MG88 and 0.2 µM 5-aza-dC).

### Example 5

### Synergistic Inhibition of Neoplastic cell Growth in Vitro

To illustrate the ability of the methods and composition of the invention to inhibit DNA MeTase and to inhibit neoplastic cell growth in a synergistic fashion, T24 bladder carcinoma cells (ATCC No. HTB-4; American Type Culture Collection, Manassas, VA) or A549 human lung carcinoma cells (ATCC No. CCL-185) were treated according to the invention and their growth pattern observed and compared to that of untreated control cells. For this purpose, one day before transfection, cells were plated onto 10 cm plates at 4 X 10⁵ cells/dish. At the time of transfection, cells were washed with phosphate buffered saline (PBS) and 5 ml of Opti-MEM media (Gibco-BRL, Mississauga, Ontario) containing 6.25 µl/ml lipofectin transfection reagent (Gibco-BRL Mississauga, Ontario) was added. The oligonucleotides used were: MG98 having the sequence 5'-UUC ATG TCA GCC AAG GCC AC-3' (SEQ ID NO:49) (this oligonucleotide is targeted to the DNA MeTase mRNA 3'UTR at nucleotides 5218 to 5199) and negative control MG207 having the sequence 5'-UUA ATG TAA CCT AAG GUC AA-3' (SEQ ID NO:3). These oligonucleotides were chemically modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of each oligonucleotide consisted of a phosphorothioate linkage between adjoining nucleotides.

The oligonucleotides were diluted to the desired concentration from a 0.1 mM stock solution in the transfection media. After a four-hour incubation at 37°C in a 5% CO₂ incubator, the plates were washed with PBS and 10 ml of fresh cell culture media was added. Following an additional two-hour incubation at 37°C, freshly prepared 5-aza-2'-deoxycytidine was added to the tissue culture medium. Cells were transfected for a total of three days and split every other day to ensure optimal transfection conditions. At the indicated time points, cells were harvested by trypsinization and pelleted by centrifugation at 1100 rpm and 4°C for five minutes. The cell pellet was resuspended in PBS and counted on a Coulter Particle Counter to determine the total cell number. Following a second centrifugation, the PBS was aspirated from the cell pellet and the pellet was frozen at 70°C. Treated and untreated cells were analyzed by counting the cells according to standard methodologies. The results of representative experiments are shown in Figs. 16, 17, and 18. Fig. 16 shows a growth curve of T24 cells that had been treated for 1-7 days with lipofectin only (diamond); 0.05µM 5-aza-dC (square); 40 nM MG98 (triangle); 0.05µM 5-aza-dC plus 40 nM MG98 (cross); 40 nM MG207 (star); or 0.05µM 5-aza-dC plus 40 nM MG207 (circle). Fig. 17 shows the number of T24 cells remaining after the cells had been treated for seven days with lipofectin only, 0.1µM 5-aza-dC, 40 nM MG207, 40 nM MG98, 0.1µM 5-aza-dC plus 40 nM MG207, or 0.1µM 5-aza-dC plus 40 nM MG98. Fig. 18 shows a growth curve of A549 cells that had been treated for 1-7 days with lipofectin only (diamond); 40 nM MG98 (square); 0.05µM 5-aza-dC plus 40 nM MG98 (triangle); 40 nM MG207 (X); or 0.05µM 5-aza-dC plus 40 nM MG207 (star). These results show that the oligonucleotides and protein effectors according to the methods of the invention are capable of inhibiting MeTase enzymatic activity and neoplastic cell growth in a synergistic fashion resulting in an increased inhibitory effect as compared to that observed using either only the oligonucleotides or only the protein effectors. The results therefore attest to the ability of the invention to inhibit DNA MeTase using effective synergistic amounts of the antisense oligonucleotide and/or of the protein effector according to the invention.

### Example 6

### Synergistic Effect on Neoplastic cells in Vivo

The purpose of this example is to illustrate the ability of the methods and compositions of the invention to treat diseases responsive to DNA MeTase inhibition in mammals. This example further provides evidence of the ability of the methods and compositions of the invention to inhibit tumor growth in a mammal. Eight to ten week old female BALB/c nude mice (Taconic Labs, Great Barrington, NY) were injected subcutaneously in the flank area with 2 x 10⁶ preconditioned Colo 205 human colon cancer cells (ATCC No. CCL-222). Preconditioning of these cells was done by a minimum of three consecutive tumor transplantations in the same strain of nude mice. Subsequently, tumor fragments of approximately 30 mgs were excised and implanted subcutaneously in mice, in the left flank area under Forene anesthesia (Abbott Labs., Geneva, Switzerland). When the tumors reached a mean volume of 100 mm³, the mice were treated intravenously for 7 consecutive days by daily bolous infusion into the tail vein, with oligonucleotide saline preparations containing 0.1-6 mg/kg of antisense oligonucleotide and/or 5-aza-dC (Sigma, St. Louis, MO) also in saline preparations according to the present invention. The optimal final concentration of the oligonucleotide is established by dose response experiments according to standard protocols. Tumor volume was calculated according to standard methods every second day post infusion (see methods, *e.g.,* in Meyer *et al*. (1989) Int. J. Cancer 43: 851-856). Treatment with the oligonucleotides and protein effectors according to the methods of the invention caused a significant reduction in tumor weight and volume relative to controls treated with saline as shown in Figs. 19, 20A, and 20B. Fig. 19 shows that animals receiving only saline (diamond) or 0.5 mg/kg MG98 (square) for seven days showed tumor volume increase while those animals receiving 0.1 mg/kg 5-aza-dC only (triangle) showed less tumor volume increase. The most dramatic reduction in tumor volume was seen in animals receiving both 0.5 mg/kg MG98 and 0.1 mg/kg 5-aza-dC (Fig. 19, X) for seven days.

Figs. 20A and 20B show the results of a similar experiment, where tumor volume is recorded for 25 days (*i.e.,* 19 additional days after final treatment. Fig. 20A shows a tumor volume growth curve versus time while Fig. 20B shows tumor volume in these mice on day 25. As shown in Fig. 20B, mice which had received both 0.5 mg/kg MG98 and 0.1 mg/kg 5-aza-dC had statistically smaller tumors (p<0.05) than mice treated with saline only or 5-aza-dC only. In addition, the activity of DNA MeTase enzyme when measured is expected to be significantly reduced relative to saline treated controls. These results show that the oligonucleotides and protein effectors according to the methods of the invention are capable of inhibiting tumor growth in a synergistic fashion.

### Example 7

### Different Synergistic Effects On Cell Cycle Progression Inhibition With Scheduling Differences

The purpose of this example is to illustrate that the synergistic effect on inhibition of cell cycle progression by a antisense oligonucleotide of the invention combined with a protein effector of the invention can be accomplished by different scheduling routines. In this example, T24 cells were used.

In Schedule A, the cells were transfected for four hours on day 0 with 75 mM MG88. The next day, they were transfected again for four hours with 75 mM MG88. The following day, they were treated with 1 µM 5-aza-dC. After twenty-four hours, the media was replaced with fresh media containing 1 µM aza-dC. For controls, the cells received either no treatment, treatment with only aza-dC (where the cells were untouched for the first two days), or treatment with only MG88 (where the cells were untouched for the third and fourth days).

In schedule B, the cells were treated with 1 µM 5-aza-dC. After twenty-four hours, the media was replaced with fresh media containing 1 µM aza-dC. Twenty-four hours later, the cells were transfected for four hours with 75 mM MG88. The next day, they were transfected again for four hours with 75 mM MG88. For controls, the cells received either no treatment, treatment with only aza-dC (where the cells were untouched for the third and fourth days), or treatment with only MG88 (where the cells were untouched for the first and second days).

On the fifth day, the cells were harvested, and fixed and permeabilized in ice-cold 70% ethanol, and stained with propidium iodide according to standard techniques (see, *e*.*g*., Ausubel *et al*, *supra;* Sambrook *et al*., *supra).* The stained cells were next analyzed by fluorescence activated cell sorter analysis (FACS analysis). According to FACS analysis of cells stained with propidium iodide, cells can typically be divided into four groups depending on the amount of DNA they possess that is stained with the propidium iodide. Typically, 4N cells (*i*.*e*., G₂/M phase cells that are about to divide) are at the farthest right of a histogram. The next set of cells with the second to highest staining are cells in S phase. The third most bright set of cells is the 2N cells in the G₁ stage of cell cycle. Finally, the least bright cells (*i*.*e*., those cells at the far left of a FACS histogram), are those that are undergoing apoptotic cell death.

As shown on Fig. 21, cells receiving both MG88 and 5-aza-dC in Schedule A (upper panel, far right) showed a decrease in the percentage of cells in the S phase of the cell cycle (27% in section M2) and increases in the percentage of apoptosing cells (16% in section M1) and cells in the S phase (22% in section M3). In contrast, cells receiving both MG88 and 5-aza-dC in Schedule B (Fig. 21, lower panel, far right) showed a more pronounced G₁ block (55% in section M2 in Fig. 21), and fewer 4N cells in the G₂/M population (13% in section M4). Notably, however, either Schedule A or Schedule B resulted in more inhibition of cell cycle progression when both MG88 and 5-aza-dC were received, as compared to cells receiving only MG88 or only 5-aza-dC (compare far right sections with middle sections in upper for Schedule A and lower panel for Schedule B).

The following examples are not part of the present invention and are for illustrative purposes only.

### Example 8

### Synergistic Effect On Inhibition Of Thymidylate Synthase With a Combination of TS Antisense Oligonucleotide and TS Protein Effector

The enzyme thymidylate synthase (TS) catalyzes a critical step in the synthesis of DNA and is especially crucial to neoplastic cells undergoing uncontrolled proliferation. The example illustrates the ability of a TS antisense oligonucleotide and a TS protein effector to act synergistically in cells to inhibit TS protein expression and inhibit the role of TS in cell cycle progression. The representative nonlimiting oligonucleotides of the inventon used in this example were MG2605, which has the following sequence: 5' UUC ATA ACC TCA GCA TUG UC 3' (SEQ ID NO:88; this oligonucleotide is targeted to the thymidylate synthase mRNA sequence provided in GenBank Accession No. X02308 at nucleotides 1267-1286) and control oligonucleotide MG2606, which has the following sequence: 5' GUC UTA AGC TCA ACA TUC UA 3' (SEQ ID NO: 81). These oligonucleotides were chemically modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of each oligonucleotide consisted of a phosphorothioate linkage between adjoining nucleotides.

The representative nonlimiting TS protein effector used in this example is the nucleoside analog 5-fluorouracil (5-FU).

To look at the ability of MG2605 to inhibit TS protein expression, T24 cells growing in culture were treated with lipofectin only (6.25 µg/ml) or lipofectin plus 10 nM, 25 nM, or 50 nM of MG2605 or MG2606 for 72 hours (*i.e.*, transfection for four hours per day for three consecutive days). After treatment, the cells were lysed and analyzed by Western blotting analysis for thymidylate synthase protein levels using a thymidylase synthase specific antibody (commercially available from Chemicon Int., Temecula, CA).

As shown in Fig. 22, T24 cells transfected for 72 hours with 25 or 50 nM MG2605, but not control MG2606 showed diminished levels of thymidylate synthase protein as compared to cells treated with lipofectin only. Equal loading of all wells is demonstrated by equivalent amounts of actin (Fig. 22).

To look at the ability of MG2605 to inhibit the role of TS in cell cycle progression, T24 cells growing in culture were treated with lipofectin only (6.25 µg/ml) or lipofectin plus 25 nM of MG2605 or MG2606, or lipofectin plus 25 nM of MG2605 or MG2606 plus 500 nM 5-FU for 72 hours (*i.e.*, transfection for four hours per day for three consecutive days, with each transfection followed by incubation with 500 nM of 5-FU). After treatment, the cells were processed for cell cycle analysis by FACS analysis as described in Example 7.

Fig. 23 shows that T24 cells treated with 500 nM 5-FU in combination with MG2605 showed a greater number of cells arrested in the S phase (section M3; bottom panel), as compared to cells treated with only MG2605 (3rd panel from top) or only 5-FU (4th panel from top).

In a similar experiment, T24 cells were transfected for four hours with 25 nM of TS antisense oligonucleotide MG2605 or control oligonucleotide MG2606 at 0 and 24 hours. After each four hour exposure to oligonucleotide, cells were returned to serum-containing media. Some of the cells were returned to serum-containing media to which was added 5 µM of 5-FU. At 72 hours, the cells were harvested, counted, and fixed and permeabilized in ice-cold 70% ethanol. The cells were then stained with propidium iodide and cell cycle analysis was performed by flow cytometry analysis as described in Example 7.

Fig. 24A shows the percentage of cells in G₁ phase (section M1), S phase (section M2), and G₂/M phase (section M3) following treatment with nothing, MG2606 only, MG2605 only, 5-FU only, MG2606 plus 5-FU, or MG2605 plus 5-FU. Treatment with both MG2605 plus 5-FU resulted in a greater number of cells arrested in the S phase of the cell cycle and fewer cells in the G₁ phase of the cell cycle as compared to treatment with either MG2605 alone or 5-FU alone (Fig. 24A). Moreover, treatment with the combination of MG2605 plus 5-FU resulted in a lower number cells as compared to the number of cells present following treatment with 5-FU only or MG2605 only (Fig. 24B).

This example demonstrates that expression of thymidylate synthase, and its role in cell cycle progression, is inhibited in T24 cells by the combination of a TS antisense oligonucleotide plus a TS protein effector to a greater degree than that seen in T24 receiving either the TS antisense oligonucleotide or the TS protein effector alone.

### Synergistic Effect On Inhibition Of HDAC Activiy With HDAC Antisense Oligonucleotide and HDAC Protein Effector

Functional histone deacetylases (HDACs) have been implicated as a requirement in cell cycle progression in both normal and neoplastic cells. The example illustrates the ability of an HDAC antisense oligonucleotide and a HDAC protein effector to act synergistically in cells to inhibit HDAC protein expression and induce the cyclin-dependent kinase inhibitor (CDKI) family, p21 ^{WAF1}.

To do this, the following representative, nonlimiting antisense oligonucleotides targeting both HDAC-1 and HDAC-2 were used: 5'- CAG CAA ATT ATG GGT CAT GCG GAU UG-3' (SEQ ID NO: 82); 5'-CAG CAA GTT ATG GGT CAT GCG GAU UG-3' (SEQ ID NO: 89); 5'- CAG CAA ATT ATG AGT CAT GCG GAU UG-3' (SEQ ID NO: 90); and 5'-CAG CAA GTT ATG AGT CAT GCG GAU UG-3' (SEQ ID NO: 83). This HDAC-1,2 antisense oligonucleotide (MG2610) was really a 1:1:1:1 mixture of each oligonucleotide (i.e., 25% of each). The control oligonucleotide (MG2637) was likewise a 1:1:1:1 mixture of the following four different oligonucleotides: 5'-AAG GAA GTC ATG GAT GAT GCG CAU UG-3' (SEQ ID NO: 84) and 5'-AAG GAA ATC ATG AAT GAT GCG CAU UG-3' (SEQ ID NO: 85). 5'-AAG GAA GTC ATG AAT GAT GCG CAU UG-3' (SEQ ID NO: 86) and 5'-AAG GAA ATC ATG GAT GAT GCG CAU UG-3' (SEQ ID NO: 87). These oligonucleotides were chemically modified as follows: A equals 2'-deoxyriboadenosine; C equals 2'-deoxyribocytidine; G equals 2'-deoxyriboguanosine; T equals 2'-deoxyribothymidine; A equals riboadenosine; U equals uridine; C equals ribocytidine; and G equals riboguanosine. The underlined bases were 2'-methoxyribose substituted nucleotides. Non-underlined bases indicate deoxyribose nucleosides. The backbone of each oligonucleotide consisted of a phosphorothioate linkage between adjoining nucleotides.

T24 cells were treated for 24 hours ( *i.e.,* 4 hours followed by incubation in fresh media for 20 hours) with lipofectin only, lipofectin plus 50 nM of MG2610 (*i*.*e*., HDAC-1,2 antisense oligonucleotide) or MG2637 control oligonucleotide, or lipofectin plus HDAC antisense oligonucleotide or control oligonucleotide plus 10 ng/ml or 25 ng/ml TSA. The treated cells were then lysed and cellular lysates analyzed by Western blotting analysis for p21^{WAF1} protein level. As shown in Fig. 25, cells treated with the combination of HDAC-1,2 antisense oligonucleotide plus TSA showed a greater increase in p21^{WAF1} protein level as compared to cells treated with either HDAC-1,2 antisense oligonucleotide or TSA alone.

This example demonstrates that treatment of cells with a combination of HDAC-1,2 antisense oligonucleotide plus an HDAC protein effector had a synergistic ability to enhance p21^{WAF1} protein levels as compared to treatment of cells with either HDAC-1,2 antisense oligonucleotide alone or HDAC protein effector alone.

### Example 10

### Synergistic Anti-Neoplastic Effect of Histone Deacetylase Antisense Oligonucleotide and HDAC Protein Effector on Tumor Cells in Vivo

The purpose of this example is to illustrate the ability of the histone deacetylase antisense oligonucleotide and the HDAC protein effector of the invention to inhibit tumor growth in a mammal. This example further provides evidence of the ability of the methods and compositions of the invention to inhibit tumor growth in domesticated mammal. Eight to ten week old female BALB/c nude mice (Taconic Labs, Great Barrington, NY) are injected subcutaneously in the flank area with 2 x 10⁶ preconditioned A549 human lung carcinoma cells. Preconditioning of these cells is done by a minimum of three consecutive tumor transplantations in the same strain of nude mice. Subsequently, tumor fragments of approximately 30 mgs are excised and implanted subcutaneously in mice, in the left flank area under Forene anesthesia (Abbott Labs., Geneva, Switzerland). When the tumors reaches a mean volume of 100 mm³, one group of mice is treated daily with oligonucleotide saline preparations containing from about 0.1 mg to about 30 mg per kg body weight of histone deacetylase antisense oligonucleotide. A second group of mice is treated daily with pharmaceutically acceptable preparations containing from about 0.01 mg to about 5 mg per kg body weight of HDAC protein effector. Yet another group of mice receives both the antisense oligonucleotide and the HDAC protein effector.

Of this third group, group A receives the antisense oligonucleotide and the HDAC protein effector together simultaneously intravenously via the tail vein. Group B receives two infusions of the antisense oligonucleotide via the tail vein on Days 1 and 2, followed on Days 3 and 4 by two intravenous infusions of the HDAC protein effector. Group C receives two infusion of the HDAC protein effector followed by two infusions of the antisense oligonucleotide on Days 3 and 4.

Control groups of mice are similarly established which receive no treatment (e.g., saline only), a mismatch antisense oligonucleotide only, a control compound that does not inhibit histone deacetylase activity, and mismatch antisense oligonucleotide with control compound.

Tumor volume is measured with calipers. Treatment with the antisense oligonucleotide plus the HDAC protein effector according to the invention causes a significant reduction in tumor weight and volume relative to controls. Preferably, the antisense oligonucleotide and the HDAC protein effector inhibit the expression and activity of the same histone deacetylase.

## Claims

1. A use of a therapeutically effective synergistic amount of an antisense oligonucleotide which inhibits expression of the gene encoding DNA methyltransferase, and a therapeutically effective synergistic amount of a moiety capable of inhibiting a product of the gene encoding DNA methyltransferase for the preparation of a pharmaceutical composition for treating cancer, beta thalassemia, sickle cell anemia, and transplantation graft rejection in a mammal wherein the composition is to be administered to a mammal, including a human, which has at least one cell affected by the disease present in its body.

2. A use of a therapeutically effective synergistic amount of an antisense oligonucleotide which inhibits expression of a gene encoding DNA methyltransferase involved in tumorigenesis, and a therapeutically effective synergistic amount of a moiety capable of inhibiting a product of the gene encoding DNA methyltransferase for the preparation of a pharmaceutical composition for inhibiting tumor growth in a mammal wherein the composition is to be administered to a mammal, including a human, which has at least one neoplastic cell present in its body.

3. The use of claim 1 or 2, wherein the antisense oligonucleotide is in operable association with a moiety capable of inhibiting a product of the gene encoding DNA methyltransferase.

4. The use of claim 3, wherein the moiety capable of inhibiting a product of the gene encoding DNA methyltransferase is selected from the group consisting of 5-aza-cytidine; 5-aza-2'-deoxycytidine, 5-fluoro-2'-deoxycytidine and 5,6-dihydro-5-azacytidine.

5. The use of claim 1 or 2, wherein the antisense oligonucleotide has at least one internucleotide linkage selected from the group consisting of phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleotide linkages.

6. The use of claim 1 or 2, wherein the antisense oligonucleotide is a chimeric oligonucleotide comprising a phosphorothioate, phosphodiester or phosphorodithioate region and an alkylphosphonate or alkylphosphonothioate region.

7. The use of claim 1 or 2, wherein the antisense oligonucleotide comprises a ribonucleotide or 2'-O-substituted ribonucleotide region and a deoxyribonucleotide region.

8. The use of claim 1, wherein said cell is to be contacted with an effective synergistic amount of at least one antisense oligonucleotide for an effective period of time.

9. The use of claim 1 or 2, wherein the mammal is to be administered a therapeutically effective synergistic amount of at least one antisense oligonucleotide for a therapeutically effective period of time.

10. The use of claim 1, wherein said cell is to be contacted with an effective synergistic amount of at least one moiety capable of inhibiting a product of the gene encoding DNA methyltransferase for an effective period of time.

11. The use of claim 1 or 2, wherein the mammal is to be administered a therapeutically effective synergistic amount of at least one moiety capable of inhibiting a product of the gene encoding DNA methyltransferase for a therapeutically effective period of time.

12. The use of claim 1, wherein each of the antisense oligonucleotide and the moiety capable of inhibiting a product of the gene encoding DNA methyltransferase is admixed with a pharmaceutically acceptable carrier prior to contacting the cell.

13. The use of claim 1 or 2, wherein each of the antisense oligonucleotide and the moiety capable of inhibiting a product of the gene encoding DNA methyltransferase is admixed with a pharmaceutically acceptable carrier prior to administration to the mammal.

14. The use of claim 1, wherein the antisense oligonucleotide and the moiety capable of inhibiting a product of the gene encoding DNA methyltransferase are mixed prior to contacting the cell.

15. The use of claim 1 or 2, wherein the antisense oligonucleotide and the moiety capable of inhibiting a product of the gene encoding DNA methyltransferase are mixed prior to administration to the mammal.

16. The use of claim 1, wherein the cell is to be contacted separately with each of the antisense oligonucleotide and the moiety capable of inhibiting a product of the gene encoding DNA methyltransferase.

17. The use of claim 16, wherein the cell is to be contacted with the antisense oligonucleotide prior to being contacted with the moiety capable of inhibiting a product of the gene encoding DNA methyltransferase

18. The use of claim 17, wherein the gene encodes a DNA methyltransferase and wherein the to be contacted cell is induced to undergo apoptosis or is arrested in the S phase of the cell cycle.

19. The use of claim 16, wherein the cell is to be contacted with the moiety capable of inhibiting a product of the gene encoding DNA methyltransferase prior to being contacted with the antisense oligonucleotide.

20. The use of claim 19, wherein the gene encodes a DNA methyltransferase and wherein the to be contacted cell is arrested in the G₁ phase of the cell cycle.

21. The use of claim 1 or 2, wherein the antisense oligonucleotide and the moiety capable of inhibiting a product of the gene encoding DNA methyltransferase are to be separately administered to the mammal.

22. The use of claim 21, wherein the antisense oligonucleotide is to be administered to the mammal prior to the administration of the moiety capable of inhibiting a product of the gene encoding DNA methyltransferase.

23. The use of claim 22, wherein the gene encodes a DNA methyltransferase and wherein the cell in the mammal to which the antisense oligonucleotide is to be administered prior to the administration of the moiety capable of inhibiting a product of the gene encoding DNA methyltransferase is induced to undergo apoptosis or is arrested in the S phase of the cell cycle.

24. The use of claim 21, wherein the moiety capable of inhibiting a product of the gene encoding DNA methyltransferase is to be administered to the mammal prior to the adminstration of the antisense oligonucleotide.

25. The use of claim 24, wherein the gene encodes a DNA methyltransferase and wherein the cell in the mammal to which the moiety capable of inhibiting a product of the gene encoding DNA methyltransferase is to be administered prior to the administration of the antisense oligonucleotide is arrested in the G₁ phase of the cell cycle.

26. The use of claim 1 or 2, wherein the gene encodes a DNA methyltransferase and wherein the cell comprises a gene whose expression has been inactivated by methylation.

27. The use of claim 26, wherein expression of the gene whose expression has been inactivated by methylation is reactivated in the contacted cell.

28. The use of claim 26, wherein expression of the gene whose expression has been inactivated by methylation is reactivated in the mammal to which has been administered the therapeutically effective synergistic amount of an antisense oligonucleotide and the therapeutically effective synergistic amount of a moiety capable of inhibiting a product of the gene encoding DNA methyltransferase.

29. The use of claim 26, wherein the gene whose expression has been inactivated by methylation is the p16^{ink4} tumor suppressor gene.

30. An inhibitor of a gene encoding DNA methyltransferase comprising an antisense oligonucleotide which inhibits expression the gene encoding DNA methyltransferase in operable association with a moiety capable of inhibiting a product of the gene encoding DNA methyltransferase of a product of the gene encoding DNA methyltransferase.

31. The inhibitor of claim 30, wherein the antisense oligonucleotide is in operable association with two or more moieties capable of inhibiting a product of the gene encoding DNA methyltransferase.

32. The inhibitor of claim 31, wherein the moiety capable of inhibiting a product of the gene encoding DNA methyltransferase is selected from the group consisting of 5-aza-cytidine, 5-aza-2'-deoxycytidine, 5-fluoro-2'-deoxycytidine and 5,6-dihydro-5-azacytidine.

33. The inhibitor of claim 30, wherein the antisense oligonucleotide has at least one internucleotide linkage selected from the group consisting of phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleotide linkages.

34. The inhibitor of claim 30, wherein the antisense oligonucleotide is a chimeric oligonucleotide comprising a phosphorothioate, phosphodiester or phosphorodithioate region and an alkylphosphonate or alkylphosphonothioate region.

35. The inhibitor of claim 30, wherein the antisense oligonucleotide comprises a ribonucleotide or 2'-O-substituted ribonucleotide region and a deoxyribonucleotide region.

36. A pharmaceutical composition comprising the inhibitor of claim 30.

37. The composition of claim 36 further comprising a pharmaceutically acceptable carrier.

## Patentansprüche

1. Verwendung einer therapeutisch wirksamen, synergistischen Menge eines Antisense-Oligonukleotids, das die Expression des Gens hemmt, das für DNA-Methyltransferase kodiert und einer therapeutisch wirksamen, synergistischen Menge eines Restes, der das Produkt eines Gens hemmen kann, das für DNA-Methyltransferase kodiert, zur Herstellung einer pharmazeutischen Zusammensetzung zur Behandlung von Krebs, β-Thalassämie, Sichelzellanämie und Transplantat-Abstoßung in einem Säuger, wobei die Zusammensetzung an den Säuger, einschließlich Menschen, zu verabreichen ist, wobei wenigstens eine Zelle im Körper des Säugers von der Krankheit betroffen ist.

2. Verwendung einer therapeutisch wirksamen, synergistischen Menge eines Antisense-Oligonukleotids, das die Expression eines Gens hemmt, das für DNA-Methyltransferase kodiert und an der Tumorentstehung beteiligt ist, und einer therapeutisch wirksamen synergistischen Menge eines Restes, der ein Produkt des Gens hemmen kann, das für DNA-Methyltransferase kodiert, zur Herstellung einer pharmazeutischen Zusammensetzung zur Hemmung von Tumorwachstum in einem Säuger, wobei die Zusammensetzung an den Säuger, einschließlich Menschen, zu verabreichen ist, wobei der Säuger in seinem Körper wenigstens eine neoplastische Zelle aufweist.

3. Verwendung gemäß Anspruch 1 oder 2, wobei das Antisense-Oligonukleotid in funktioneller Assoziation mit einem Rest steht, der das Produkt eines Gens hemmen kann, das für DNA-Methyltransferase kodiert.

4. Verwendung gemäß Anspruch 3, wobei der Rest, der das Produkt eines Gens hemmen kann, das für DNA-Methyltransferase kodiert, ausgewählt ist aus der Gruppe bestehend aus 5-aza-cytidin, 5-aza-2'-desoxycytidin, 5-fluor-2'-desoxycytidin und 5,6-dihydro-5-azacytidin.

5. Verwendung gemäß Anspruch 1 oder 2, wobei das Antisense-Oligonukleotid wenigstens eine Internukleotidverknüpfung besitzt, ausgewählt aus der Gruppe der Internukleotidverknüpfungen bestehend aus Phosphorthioat, Phosphordithioat, Alkylphosphonat, Alkylphosphonothioat, Phosphotriester, Phosphoramidat, Siloxan, Carbonat, Carboxymethylester, Acetamidat, Carbamat, Thioether, überbrücktes Phosphoramidat, überbrücktes Methylenphosphonat, überbrücktes Phosphorthioat und Sulfon.

6. Verwendung gemäß Anspruch 1 oder 2, wobei das Antisense-Oligonukleotid ein chimäres Oligonukleotid ist, umfassend einen Phosphorthioat-, einen Phosphodiester- oder Phosphordithioatbereich und einen Alkylphosphonat- oder einen Alkylphosphonothioatbereich.

7. Verwendung gemäß Anspruch 1 oder 2, wobei das Antisense-Oligonukleotid einen Ribonukleotid- oder einen 2'-O-substituierten Ribonukleotidbereich und einen Desoxyribonukleotidbereich umfasst.

8. Verwendung gemäß Anspruch 1, wobei die Zelle mit einer wirksamen, synergistischen Menge wenigstens eines Antisense-Oligonukleotids für einen wirksamen Zeitraum in Kontakt zu bringen ist.

9. Verwendung gemäß Anspruch 1 oder 2, wobei eine therapeutisch wirksame, synergistische Menge wenigstens eines Antisense-Oligonukleotids für einen therapeutisch wirksamen Zeitraum an einen Säuger zu verabreichen ist.

10. Verwendung gemäß Anspruch 1, wobei die Zelle mit einer wirksamen synergistischen Menge wenigstens eines Restes, der das Produkt eines Gens hemmen kann, das für DNA-Methyltransferase kodiert, für einen wirksamen Zeitraum in Kontakt zu bringen ist.

11. Verwendung gemäß Anspruch 1 oder 2, wobei eine therapeutisch wirksame, synergistische Menge wenigstens eines Restes, der das Produkt eines Gens hemmen kann, das für DNA-Methyltransferase kodiert, für einen therapeutisch wirksamen Zeitraum an den Säuger zu verabreichen ist.

12. Verwendung gemäß Anspruch 1, wobei sowohl das Antisense-Oligonukleotid als auch der Rest, der ein Produkt des Gens hemmen kann, das für DNA-Methyltransferase kodiert, vor dem In-Kontakt-bringen mit der Zelle mit einem pharmazeutisch verträglichen Träger vermischt werden.

13. Verwendung gemäß Anspruch 1 oder 2, wobei sowohl das Antisense-Oligonukleotid als auch der Rest, der ein Produkt des Gens hemmen kann, das für DNA-Methyltransferase kodiert, vor dem Verabreichen an den Säuger mit einem pharmazeutisch verträglichen Träger vermischt werden.

14. Verwendung gemäß Anspruch 1, wobei das Antisense-Oligonukleotid und der Rest, der ein Produkt des Gens hemmen kann, das für DNA-Methyltransferase kodiert, vor dem In-Kontakt-bringen mit der Zelle vermischt werden.

15. Verwendung gemäß Anspruch 1 oder 2, wobei das Antisense-Oligonukleotid und der Rest, der ein Produkt des Gens hemmen kann, das für DNA-Methyltransferase kodiert, vor dem Verabreichen an den Säuger vermischt werden.

16. Verwendung gemäß Anspruch 1, wobei die Zelle sowohl mit dem Antisense-Oligonukleotid, als auch mit dem Rest, der ein Produkt des Gens hemmen kann, das für DNA-Methyltransferase kodiert, getrennt in Kontakt zu bringen ist.

17. Verwendung gemäß Anspruch 16, wobei die Zelle mit dem Antisense-Oligonukleotid vor dem Rest, der ein Produkt des Gens hemmen kann, das für DNA-Methyltransferase kodiert, in Kontakt zu bringen ist.

18. Verwendung gemäß Anspruch 17, wobei das Gen für eine DNA-Methyltransferase kodiert und wobei die in Kontakt zu bringende Zelle zur Apoptose induziert wird oder in der S-Phase des Zellzyklus arretiert ist.

19. Verwendung gemäß Anspruch 16, wobei die Zelle mit dem Rest, der ein Produkt des Gens hemmen kann, das für DNA-Methyltransferase kodiert, vor dem Antisense-Oligonukleotid in Kontakt zu bringen ist.

20. Verwendung gemäß Anspruch 19, wobei das Gen für eine DNA-Methyltransferase kodiert und wobei die in Kontakt zu bringende Zelle in der G1-Phase des Zellzyklus arretiert ist.

21. Verwendung gemäß Anspruch 1 oder 2, wobei das Antisense-Oligonukleotid und der Rest, der ein Produkt des Gens hemmen kann, das für DNA-Methyltransferase kodiert, getrennt an den Säuger zu verabreichen sind.

22. Verwendung gemäß Anspruch 21, wobei das Antisense-Oligonukleotid vor dem Rest, der ein Produkt des Gens hemmen kann, das für DNA-Methyltransferase kodiert, an den Säuger zu verabreichen ist.

23. Verwendung gemäß Anspruch 22, wobei das Gen für eine DNA-Methyltransferase kodiert und wobei die Zelle in dem Säuger, an den das Antisense-Oligonukleotid vor der Verabreichung des Restes, der ein Produkt eines Gens hemmen kann, das für DNA-Methyltransferase kodiert, zu verabreichen ist, zur Apoptose induziert wird oder in der S-Phase des Zellzyklus arretiert ist.

24. Verwendung gemäß Anspruch 21, wobei der Rest, der ein Produkt des Gens hemmen kann, das für DNA-Methyltransferase kodiert, vor dem Antisense-Oligonukleotid an den Säuger zu verabreichen ist.

25. Verwendung gemäß Anspruch 24, wobei das Gen für eine DNA-Methyltransferase kodiert und wobei die Zelle in dem Säuger, an den der Rest, der ein Produkt eines Gens hemmen kann, das für DNA-Methyltransferase kodiert, vor dem Antisense-Oligonukleotid zu verabreichen ist, in der G1-Phase des Zellzyklus arretiert ist.

26. Verwendung gemäß Anspruch 1 oder 2, wobei das Gen für eine DNA-Methyltransferase kodiert und wobei die Zelle ein Gen umfasst, dessen Expression durch Methylierung inaktiviert wurde.

27. Verwendung gemäß Anspruch 26, wobei die Expression des Gens, dessen Expression durch Methylierung inaktiviert wurde, in der kontaktierten Zelle reaktiviert wurde.

28. Verwendung gemäß Anspruch 26, wobei die Expression des Gens, dessen Expression durch Methylierung inaktiviert wurde, in dem Säuger reaktiviert wird, an den die therapeutisch wirksame, synergistische Menge eines Antisense-Oligonukleotids und die therapeutische wirksame, synergistische Menge eines Restes, der ein Produkt eines Gens hemmen kann, wobei das Gen für DNA-Methyltransferase kodiert, verabreicht wurde.

29. Verwendung gemäß Anspruch 26, wobei das Gen, dessen Expression durch Methylierung inaktiviert wurde, das Tumorsuppressorgen p16^{ink4} ist.

30. Inhibitor eines Gens, das für DNA-Methyltransferase kodiert, umfassend ein Antisense-Oligonukleotid, das die Expression des Gens hemmt, das für DNA-Methyltransferase kodiert, in funktioneller Assoziation mit einem Rest, der ein Produkt eines Gens hemmen kann, das für DNA-Methyltransferase kodiert.

31. Inhibitor gemäß Anspruch 30, wobei das Antisense-Oligonukleotid in funktioneller Assoziation mit zwei oder mehreren Resten steht, die ein Produkt des Gens hemmen können, das für DNA-Methyltransferase kodiert.

32. Inhibitor gemäß Anspruch 31, wobei der Rest, der ein Produkt des Gens hemmen kann, das für DNA-Methyltransferase kodiert, ausgewählt ist aus der Gruppe bestehend aus 5-aza-cytidin, 5-aza-2'-desoxycytidin, 5-fluor-2'-desoxycytidin und 5,6-dihydro-5-azacytidin.

33. Inhibitor gemäß Anspruch 30, wobei das Antisense-Oligonukleotid wenigstens eine Internukleotidverknüpfung besitzt, ausgewählt aus der Gruppe der Internukleotidverknüpfungen bestehend aus Phosphorthioat, Phosphordithioat, Alkylphosphonat, Alkylphosphonothioat, Phosphotriester, Phosphoramidat, Siloxan, Carbonat, Carboxymethylester, Acetamidat, Carbamat, Thioether, überbrücktes Phosphoramidat, überbrücktes Methylenphosphonat, überbrücktes Phosphorthioat und Sulfon.

34. Inhibitor gemäß Anspruch 30, wobei das Antisense-Oligonukleotid ein chimäres Oligonukleotid ist, umfassend einen Phosphorthioat-, Phosphodiester- oder Phosphordithioatbereich und einen Alkylphosphonat- oder Alkylphosphonothioatbereich.

35. Inhibitor gemäß Anspruch 30, wobei das Antisense-Oligonukleotid einen Ribonukleotid- oder einen 2'-O-substituierten Ribonukleotidbereich und einen Desoxyribonukleotidbereich umfasst.

36. Pharmazeutische Zusammensetzung, umfassend den Inhibitor gemäß Anspruch 30.

37. Zusammensetzung gemäß Anspruch 36, weiter umfassend einen pharmazeutisch verträglichen Träger.

## Revendications

1. Utilisation d'une quantité synergique efficace du point de vue thérapeutique d'un oligonucléotide antisens qui inhibe l'expression du gène codant pour l'ADN méthyltransférase, et d'une quantité synergique efficace du point de vue thérapeutique d'une entité capable d'inhiber un produit du gène codant pour l'ADN méthyltransférase pour la préparation d'une composition pharmaceutique pour le traitement de cancer, thalassémie bêta, anémie à hématies falciformes, et rejet de greffe de transplantation chez un mammifère, tandis que la composition est destinée à être administrée à un mammifère, y compris un humain, qui a au moins une cellule affectée par la maladie présente dans son corps.

2. Utilisation d'une quantité synergique efficace du point de vue thérapeutique d'un oligonucléotide antisens qui inhibe l'expression d'un gène codant pour l'ADN méthyltransférase impliqué dans la tumorigenèse, et d'une quantité synergique efficace du point de vue thérapeutique d'une entité capable d'inhiber un produit du gène codant pour l'ADN méthyltransférase pour la préparation d'une composition pharmaceutique pour l'inhibition de la croissance des tumeurs chez un mammifère, tandis que la composition est destinée à être administrée à un mammifère, y compris un humain, qui a au moins une cellule néoplasique dans son corps.

3. Utilisation selon la revendication 1 ou 2, dans laquelle l'oligonucléotide antisens est en association apte à être opérationnelle avec une entité capable d'inhiber un produit du gène codant pour l'ADN méthyltransférase.

4. Utilisation selon la revendication 3, dans laquelle l'entité capable d'inhiber un produit du gène codant pour l'ADN méthyltransférase est choisie dans le groupe consistant en 5-aza-cytidine, 5-aza-2'-désoxycytidine, 5-fluoro-2'-désoxycytidine et 5,6-dihydro-5-azacytidine.

5. Utilisation selon la revendication 1 ou 2, dans laquelle l'oligonucléotide antisens a au moins une liaison internucléotidique choisie dans le groupe consistant en des liaisons intemucléotidiques phosphorothioate, phosphorodithioate, alkylphosphonate, alkylphosphonothioate, phosphotriester, phosphoramidate, siloxane, carbonate, ester carboxyméthylique, acétamidate, carbamate, thioéther, phosphoramidate ponté, méthylène phosphonate ponté, phosphorothioate ponté et sulfone.

6. Utilisation selon la revendication 1 ou 2, dans laquelle l'oligonucléotide antisens est un oligonucléotide chimère comprenant une région phosphorothioate, phosphodiester ou phosphorodithioate et une région alkylphosphonate ou alkylphosphonothioate.

7. Utilisation selon la revendication 1 ou 2, dans laquelle l'oligonucléotide antisens comprend une région de ribonucléotide ou de ribonucléotide 2'-O-substitué et une région de désoxyribonucléotide.

8. Utilisation selon la revendication 1, dans laquelle ladite cellule est destinée à être mise en contact avec une quantité synergique efficace d'au moins un oligonucléotide antisens pendant une période de temps efficace.

9. Utilisation selon la revendication 1 ou 2, dans laquelle le mammifère est destiné à recevoir l'administration d'une quantité synergique efficace du point de vue thérapeutique d'au moins un oligonucléotide antisens pendant une période de temps efficace du point de vue thérapeutique.

10. Utilisation selon la revendication 1, dans laquelle ladite cellule est destinée à être mise en contact avec une quantité synergique efficace d'au moins une entité capable d'inhiber un produit du gène codant pour l'ADN méthyltransférase pendant une période de temps efficace.

11. Utilisation selon la revendication 1 ou 2, dans laquelle le mammifère est destiné à recevoir l'administration d'une quantité synergique efficace du point de vue thérapeutique d'au moins une entité capable d'inhiber un produit du gène codant pour l'ADN méthyltransférase pendant une période de temps efficace du point de vue thérapeutique.

12. Utilisation selon la revendication 1, dans laquelle chacun parmi l'oligonucléotide antisens et l'entité capable d'inhiber un produit du gène codant pour l'ADN méthyltransférase est mélangé avec un support pharmaceutiquement acceptable avant d'être en contact avec la cellule.

13. Utilisation selon la revendication 1 ou 2, dans laquelle chacun parmi l'oligonucléotide antisens et l'entité capable d'inhiber un produit du gène codant pour l'ADN méthyltransférase est mélangé avec un support pharmaceutiquement acceptable avant administration au mammifère.

14. Utilisation selon la revendication 1, dans laquelle l'oligonucléotide antisens et l'entité capable d'inhiber un produit du gène codant pour l'ADN méthyltransférase sont mélangés avant d'être en contact avec la cellule.

15. Utilisation selon la revendication 1 ou 2, dans laquelle l'oligonucléotide antisens et l'entité capable d'inhiber un produit du gène codant pour l'ADN méthyltransférase sont mélangés avant administration au mammifère.

16. Utilisation selon la revendication 1, dans laquelle la cellule est destinée à être mise en contact séparément avec chacun parmi l'oligonucléotide antisens et l'entité capable d'inhiber un produit du gène codant pour l'ADN méthyltransférase.

17. Utilisation selon la revendication 16, dans laquelle la cellule est destinée à. être mise en contact avec l'oligonucléotide antisens avant d'être mise en contact avec l'entité capable d'inhiber un produit du gène codant pour l'ADN méthyltransférase.

18. Utilisation selon la revendication 17, dans laquelle le gène code pour une ADN méthyltransférase, et où la cellule destinée à être mise en contact est induite à subir une apoptose ou est arrêtée dans la phase S du cycle cellulaire.

19. Utilisation selon la revendication 16, dans laquelle la cellule est destinée à être mise en contact avec l'entité capable d'inhiber un produit du gène codant pour l'ADN méthyltransférase avant d'être mise en contact avec l'oligonucléotide antisens.

20. Utilisation selon la revendication 19, dans laquelle le gène code pour une ADN méthyltransférase, et où la cellule destinée à être mise en contact est arrêtée dans la phase G₁ du cycle cellulaire.

21. Utilisation selon la revendication 1 ou 2, dans laquelle l'oligonucléotide antisens et l'entité capable d'inhiber un produit du gène codant pour l'ADN méthyltransférase sont destinés à être administrés séparément au mammifère.

22. Utilisation selon la revendication 21, dans laquelle l'oligonucléotide antisens est destiné à être administré au mammifère avant l'administration de l'entité capable d'inhiber un produit du gène codant pour l'ADN méthyltransférase.

23. Utilisation selon la revendication 22, dans laquelle le gène code pour une ADN méthyltransférase, et où la cellule dans le mammifère auquel l'oligonucléotide antisens est destiné à être administré avant l'administration de l'entité capable d'inhiber un produit du gène codant pour l'ADN méthyltransférase est induit à subir une apoptose ou est arrêté dans la phase S du cycle cellulaire.

24. Utilisation selon la revendication 21, dans laquelle l'entité capable d'inhiber un produit du gène codant pour l'ADN méthyltransférase est destinée à être administrée au mammifère avant l'administration de l'oligonucléotide antisens.

25. Utilisation selon la revendication 24, dans laquelle le gène code pour une ADN méthyltransférase, et où la cellule dans le mammifère auquel l'entité capable d'inhiber un produit du gène codant pour l'ADN méthyltransférase est destinée à être administrée avant l'administration de l'oligonucléotide antisens est arrêtée dans la phase G₁ du cycle cellulaire.

26. Utilisation selon la revendication 1 ou 2, dans laquelle le gène code pour une ADN méthyltransférase et où la cellule comprend un gène dont l'expression a été inactivée par méthylation.

27. Utilisation selon la revendication 26, dans laquelle l'expression du gène dont l'expression a été inactivée par méthylation est réactivée dans la cellule contactée.

28. Utilisation selon la revendication 26, dans laquelle l'expression du gène dont l'expression a été inactivée par méthylation est réactivée dans le mammifère auquel a été administrée la quantité synergique efficace du point de vue thérapeutique d'un oligonucléotide antisens et la quantité synergique efficace du point de vue thérapeutique d'une entité capable d'inhiber un produit du gène codant pour l'ADN méthyltransférase.

29. Utilisation selon la revendication 26, dans laquelle le gène dont l'expression a été inactivée par méthylation est le gène suppresseur de tumeurs p16^{ink4}.

30. Inhibiteur d'un gène codant pour l'ADN méthyltransférase, comprenant un oligonucléotide antisens qui inhibe l'expression du gène codant pour l'ADN méthyltransférase en association apte à être opérationnelle avec une entité capable d'inhiber un produit du gène codant pour l'ADN méthyltransférase.

31. Inhibiteur selon la revendication 30, dans lequel l'oligonucléotide antisens est en association apte à être opérationnelle avec deux ou plus de deux entités capables d'inhiber un produit du gène codant pour l'ADN méthyltransférase.

32. Inhibiteur selon la revendication 31, dans lequel l'entité capable d'inhiber un produit du gène codant pour l'ADN méthyltransférase est choisie dans le groupe consistant en 5-aza-cytidine, 5-aza-2'-désoxycytidine, 5-fluoro-2'-désoxycytidine et 5,6-dihydro-5-azacytidine.

33. Inhibiteur selon la revendication 30, dans lequel l'oligonucléotide antisens a au moins une liaison intemucléotidique choisie dans le groupe consistant en les liaisons internucléotidiques phosphorothioate, phosphorodithioate, alkylphosphonate, alkylphosphonothioate, phosphotriester, phosphoramidate, siloxane, carbonate, ester carboxyméthylique, acétamidate, carbamate, thioéther, phosphoramidate ponté, méthylène phosphonate ponté, phosphorothioate ponté et sulfone.

34. Inhibiteur selon la revendication 30, dans lequel l'oligonucléotide antisens est un oligonucléotide chimère comprenant une région phosphorothioate, phosphodiester ou phosphorodithioate et une région alkylphosphonate ou alkylphosphonothioate.

35. Inhibiteur selon la revendication 30, dans lequel l'oligonucléotide antisens comprend une région de ribonucléotide ou de ribonucléotide 2'-O-substitué et une région de désoxyribonucléotide.

36. Composition pharmaceutique comprenant l'inhibiteur selon la revendication 30.

37. Composition selon la revendication 36, comprenant en outre un support pharmaceutiquement acceptable.
